# EUROPÄISCHE PATENTSCHRIFT

(11) **EP 2 036 880 B1**
(45) Veröffentlichungstag und Bekanntmachung des Hinweises auf die Patenterteilung: **22.01.2014**
(21) Anmeldenummer: 08015440.4
(22) Anmeldetag: 02.09.2008
(51) Int. Cl.: C07C 68/06

(54) **Verfahren zur Herstellung von Diaryl- oder Alkylarylcarbonaten aus Dialkylcarbonaten**
Method for producing diarylcarbonates or arylalkylcarbonates from dialkylcarbonates
Procédé de fabrication de carbonates de diaryle ou d'alkylaryle à partir de carbonates de dialkyle

(30) Priorität: 14.09.2007 DE 102007044033
(43) Veröffentlichungstag der Anmeldung: 18.03.2009
(73) Patentinhaber: Bayer Intellectual Property GmbH, 40789 Monheim (DE)
(72) Erfinder: Düx, Andre, 50321 Brühl (DE); Ooms, Pieter, Dr., 47800 Krefeld (DE); Rechner, Johann, Dr., 47906 Kempen (DE); Böhm, Matthias, 51373 Leverkusen (DE); Hallenberger, Kaspar, 51375 Leverkusen (DE); Ronge, Georg, Dr., 40549 Düsseldorf (DE); Eynde, Vanden, Johan, 9052 Zwijnaarde (Gent) (BE)
(74) Vertreter: BIP Patents

(56) Entgegenhaltungen:
- EP-A1- 0 781 760
- EP-A1- 1 795 522
- AGRAWAL, R.; FIDKOWSKI, Z. T.: "On the Use of Intermediate Reboilers in the Rectifying Section and Condensers in the Stripping Section of a Distillation Column" IND. ENG. CHEM. RES., Bd. 35, 1996, Seiten 2801-2807, XP002578159
- D. STEINMEYER: "Process Energy Conservation", KIRK-OTHMER ENCYCLOPEDIA OF CHEMICAL TECHNOLOGY, 4 December 2000 (2000-12-04), pages 1-27, DOI: 10.1002/0471238961.161815031920050

## Beschreibung

Gegenstand der Erfindung ist ein Verfahren zur Herstellung von Diarylcarbonaten und/oder Alkylarylcarbonaten aus Dialkylcarbonaten und aromatischen Hydroxyverbindungen unter Einsatz von wenigstens zwei Reaktionskolonnen, wobei die Kondensationswärme einer oder mehrerer dieser Kolonnen in anderen Verfahrensschritten dieses Verfahrens genutzt wird.

Die Herstellung von aromatischen und aliphatisch-aromatischen Kohlensäurestern (Carbonaten) durch Umesterung ausgehend von aliphatischen Kohlensäurestern und aromatischen Hydroxyverbindungen ist im Prinzip bekannt. Dabei handelt es sich um eine Gleichgewichtsreaktion, wobei die Lage des Gleichgewichts fast vollständig in Richtung der aliphatisch substituierten Carbonate verschoben ist. Daher ist es verhältnismäßig leicht, aus aromatischen Carbonaten und Alkoholen aliphatische Carbonate herzustellen. Um die Reaktion jedoch im umgekehrten Sinne in Richtung aromatischer Carbonate durchzuführen, ist es notwendig, das sehr ungünstig liegende Gleichgewicht effektiv auf die Seite der aromatischen Carbonate zu verschieben, wobei nicht nur sehr aktive Katalysatoren, sondern auch geeignete Verfahrensführungen zur Anwendung gelangen müssen.

Es ist bekannt, derartige Gleichgewichtsreaktionen in Kolonnen durchzuführen und sie auf diese Weise vorteilhaft in Richtung der gewünschten Produktbildung zu verschieben (z.B. U. Block, Chem.-Ing. Techn. 49, 151 (1977); DE-OS 38 09 417; B. Schleper, B. Gutsche, J. Wnuck und L.Jeromin, Chem.-Ing.-Techn. 62, 226 (1990); Ullmans Encyclopädie der techn. Chemie, 4. Aufl., Bd. 3; S375 ff. 1973).

In den bekannten Verfahren erfolgt die Umesterung daher auch vorzugsweise kontinuierlich im Sinne einer Gegenstromumesterung in einer oder mehreren Reaktionskolonnen.

In EP-A 0 461 274 wird ein kontinuierlicher Umesterungsprozess zur Herstellung von aromatischen Carbonaten in einer oder in mehreren hintereinander geschalteten mehrstufigen Kolonnen beschrieben, wobei Dialkylcarbonate oder Alkylarylcarbonate mit Phenolen umgesetzt werden und am Kopf der Kolonnen die leichtflüchtigen Produkte, nämlich die Reaktionsalkohole und Dialkylcarbonate und am Sumpf der Kolonnen die schwersiedenden Produkte, wie z.B. Diarylcarbonate entnommen werden. Es werden jedoch keine Hinweise gegeben inwiefern man die bei diesem Verfahren anfallende Abwärme nutzen kann.

DE-A 42 26 756 beschreibt ein zweistufiges Verfahren zur Herstellung von Diarylcarbonaten durch Umesterung eines Dialkylcarbonates mit einer aromatischen Hydroxyverbindung, bei dem in einer ersten Stufe aus den Ausgangsstoffen zunächst das korrespondierende Alkylarylcarbonat und in einer zweiten Stufe das Diarylcarbonat gebildet wird. Die Angaben in der Verfahrensbeschreibung beschränken sich dabei auf die Reaktionsbedingungen, den verwendeten Katalysator und die Ausführung der Reaktionskolonnen. Es werden jedoch keine Angaben zur Nutzung anfallender Abwärme gemacht.

DE-A 42 26 755 beschreibt ein Verfahren zur Herstellung von Diarylcarbonaten in zwei energetisch und stofflich miteinander gekoppelten Reaktionskolonnen, wobei in der ersten Stufe eine aromatische Hydroxyverbindungen und ein Dialkylcarbonat, zur Reaktion gebracht werden und das dabei gebildete Alkylarylcarbonat in der zweiten Stufe entweder durch Umesterung mit der aromatischen Hydroxyverbindung oder durch Disproportionierung zum Diarylcarbonat umgesetzt wird. Problematisch ist hierbei allerdings, dass durch die stoffliche und energetische Integration des Verfahrens die Reaktionsbedingungen für die Bildung des Alkylaryl- bzw. Diarylcarbonats nicht optimal gewählt werden können, da diese durch den in beiden Schritten vorliegenden und nahezu identischen Druck festgelegt sind.

Die EP-A 781 760 beschreibt ein kontinuierliches Verfahren zur Herstellung aromatischer Carbonate durch Reaktion eines Dialkylcarbonates mit einer aromatischen Hydroxyverbindung in Anwesenheit eines Katalysators, kontinuierlicher Entfernung des bei der Reaktion entstehenden aromatischen Carbonats, der alkoholischen Nebenprodukte, des Dialkylcarbonats und der aromatischen Hydroxyverbindung, wobei das Dialkylcarbonat und die aromatische Hydroxyverbindung wieder in die Reaktion zurückgeführt werden. Zwar sind die beschriebenen Verfahrensschritte effektiv bezüglich der Reaktionsführung im Sinne einer hohen Raum-ZeitAusbeute und der Aufarbeitung im Sinne einer möglichst effizienten Trennsequenz, jedoch zeigt das Verfahren keinerlei Möglichkeiten für eine energetische Integration der Reaktion und Aufarbeitungsschritte.

WO-A 2006/001256 beschreibt ein Verfahren, bei dem eine aromatische Hydroxyverbindung mit einem Dialkylcarbonat in Anwesenheit eines Katalysators umgesetzt wird sowie eine hierzu geeignete technische Vorrichtung. Auch hier werden keine Anhaltspunkte für energetische Integration gegeben.

Ohne entsprechend effiziente Energieintegration ist der Energieverbrauch der vorangehend beschriebenen Verfahren bekanntermaßen hoch, was wiederum die Vorteilhaftigkeit der phosgenfreien Herstellung von Arylcarbonaten aus ökologischer und ökonomischer Hinsicht in Frage stellt.

WO-A 2004/016577 beschreibt ein Verfahren zur Herstellung von aromatischen Carbonaten aus Dialkylcarbonat und einer aromatischen Hydroxyverbindung in Gegenwart eines Katalysators in mehreren getrennten und in Serie geschalteten Reaktionszonen einer Reaktoranordnung, wobei die anfallende Kondensationswärme bei der Kondensation des Dampfstromes der letzten Reaktionszone zur Erwärmung des in die erste Reaktionszone eingeleiteten Flüssigkeitsstromes verwendet wird. Nachteilig an diesem Verfahren ist jedoch die aufwändige Reaktoranordnung. Zudem ist die energetische Integration dieses Verfahrens verbesserungswürdig.

JP-A 2002-020351 beschreibt ein diskontinuierliches Verfahren zur Herstellung von Diarylcarbonat, aus dem Wärme zur Dampferzeugung genutzt werden kann. Nachteilig an diesem Verfahren sind jedoch die diskontinuierliche Durchführung sowie die zur Umsetzung verwendete Reaktoranordnung mit aufgesetzter Destillationskolonne. Nachteilig an diesem Verfahren ist jedoch insbesondere dessen diskontinuierliche Fahrweise.

EP 1 795 522 A1 offenbart ein Verfahren zur Herstellung eines Diarylcarbonats aus einem Dialkylcarbonat und einer aromatischen Hydroxyverbindung, wobei der am Kopf einer ersten Reaktionskolonne (Kolonne A) anfallende Dampf einem Kondensator zugeführt und die dort gewonnene Wärme zur Aufheizung eines Eduktstroms verwendet wird. Die dort verfügbaren Energieniveaus sind jedoch nicht für eine Energieintegration gemäß vorliegendem Anspruch möglich.

AGRAWAL, R.; FIDKOWSKI, Z. T.: "On the Use of Intermediate Reboilers in the Rectifying Section and Condensers in the Stripping Section of a Distillation Column" IND. ENG. CHEM. RES., Bd. 35, 1996, Seiten 2801-2807 beschreibt eine Destillation mit der Verwendung eines Zwischenkondensators im Verstärkungsteil der ersten Reaktionskolonne. Diese Lehre kann jedoch nicht ohne weiteres auf eine Reaktionskolonne übertragen werden, da die hier erfolgenden Reaktionen grundverschieden sind.

Es bestand demnach weiterhin Bedarf, ein Verfahren zur Herstellung von aromatischen Carbonaten, d.h. Diaryl- und/oder Alkylarylcarbonaten, vorzugsweise Diarylcarbonaten, bereitzustellen, welches die vorangehend genannten Nachteile nicht aufweist und in welchem gegenüber den vorangehend genannten bekannten Verfahren eine Energieintegration in effizienter Weise möglich ist bzw. eine verbesserte Energieintegration erreicht werden kann.

Die Aufgabe, die der Erfindung zugrunde lag, bestand demnach darin, ein Verfahren zur Herstellung von aromatischen Carbonaten, d.h. Diaryl- und/oder Alkylarylcarbonaten, vorzugsweise Diarylcarbonaten, bereitzustellen, in welchem gegenüber bekannten Verfahren eine Energieintegration in effizienter Weise möglich ist bzw. eine verbesserte Energieintegration erreicht werden kann.

Diese Aufgabe wurde erfindungsgemäß dadurch gelöst, dass durch geeignete Wärmeintegration in einem Verfahren zur Herstellung von aromatischen Carbonaten, d.h. Diaryl- und/oder Alkylarylcarbonaten, aus Dialkylcarbonaten und aromatischen Hydroxyverbindungen in wenigstens zwei Reaktionskolonnen der Energieverbrauch deutlich reduziert werden kann.

Die Erfindung betrifft ein Verfahren zur Herstellung von wenigstens einem Diarylcarbonat und/oder Alkylarylcarbonat aus wenigstens einem Dialkylcarbonat und wenigstens einer aromatischen Hydroxyverbindung gemäß Anspruch 1.

Im Rahmen der Erfindung hergestellte Diarylcarbonate sind bevorzugt solche der allgemeinen Formel (I) 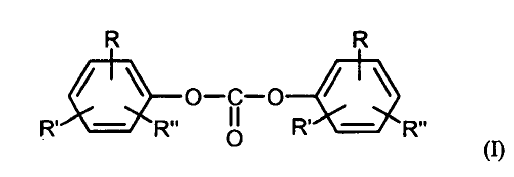 wobei R, R' und R" unabhängig voneinander H, lineares oder verzweigtes, gegebenenfalls substituiertes C₁-C₃₄-Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl, C₆-C₃₄-Aryl oder einen Halogenrest, bevorzugt einen Chlorrest darstellen und R, R' und R" auf beiden Seiten der Formel (I) gleich oder verschieden sein können. R kann auch -COO-R''' bedeuten, wobei R"' H, gegebenenfalls verzweigtes C₁-C₃₄ Alkyl, bevorzugt C₁-C₆-Alkyl, besonders bevorzugt C₁-C₄-Alkyl, C₁-C₃₄-Alkoxy, bevorzugt C₁-C₆-Alkoxy, besonders bevorzugt C₁-C₄-Alkoxy, C₅-C₃₄-Cycloalkyl, C₇-C₃₄-Alkylaryl oder C₆-C₃₄-Aryl sein kann. Bevorzugt sind R, R' und R" auf beiden Seiten der Formel (I) gleich. Ganz besonders bevorzugt stehen R, R' und R" für H.

Diarylcarbonate der allgemeinen Formel (I) sind beispielsweise: Diphenylcarbonat, Methylphenylphenyl-carbonate und Di-(methylphenyl)-carbonate, auch als Gemisch, wobei die Stellung der Methylgruppe an den Phenylringen beliebig sein kann, sowie Dimethylphenyl-phenyl-carbonate und Di-(dimethylphenyl)-carbonate, auch als Gemisch, wobei die Stellung der Methylgruppen an den Phenylringen beliebig sein kann, Chlorphenyl-phenyl-carbonate und Di-(chlorphenyl)-carbonate, wobei die Stellung der Methylgruppe an den Phenylringen beliebig sein kann, 4-Ethylphenyl-phenyl-carbonat, Di-(4-ethylphenyl)-carbonat, 4-n-Propylphenyl-phenyl-carbonat, Di-(4-n-propylphenyl)-carbonat, 4-iso-Propylphenyl-phenyl-carbonat, Di-(4-iso-propylphenyl)-carbonat, 4-n-Butylphenyl-phenyl-carbonat, Di-(4-n-butylphenyl)-carbonat, 4-iso-Butylphenyl-phenyl-carbonat, Di-(4-iso-butylphenyl)-carbonat, 4-tert-Butylphenyl-phenyl-carbonat, Di-(4-tert-butylphenyl)-carbonat, 4-n-Pentylphenyl-phenyl-carbonat, Di-(4-n-pentylphenyl)-carbonat, 4-n-Hexylphenyl-phenyl-carbonat, Di-(4-n-hexylphenyl)-carbonat, 4-iso-Octylphenyl-phenyl-carbonat, Di-(4-iso-octylphenyl)-carbonat,4-n-Nonylphenyl-phenyl-carbonat, Di-(4-n-nonylphenyl)-carbonat, 4-Cyclohexylphenyl-phenyl-carbonat, Di-(4-cyclohexylphenyl)-carbonat, 4-(1-Methyl-1-phenylethyl)-phenyl-phenyl-carbonat, Di-[4-(1-methyl-1-phenylethyl)-phenyl]-carbonat, Biphenyl-4-yl-phenyl-carbonat, Di-(biphenyl-4-yl)-carbonat, (1-Naphthyl)-phenyl-carbonat, (2-Naphthyl)-phenyl-carbonat, Di-(1-naphthyl)-carbonat, Di-(2-naphthyl)-carbonat, 4-(1-Naphthyl)-phenyl-phenyl-carbonat, 4-(2-Naphthyl)-phenyl-phenyl-carbonat, Di-[4-(1-naphthyl)phenyl]-carbonat, Di-[4-(2-naphthyl)phenyl]-carbonat, 4-Phenoxyphenyl-phenyl-carbonat, Di-(4-phenoxyphenyl)-carbonat, 3-Pentadecylphenyl-phenyl-carbonat, Di-(3-pentadecylphenyl)-carbonat, 4-Tritylphenylphenyl-carbonat, Di-(4-tritylphenyl)-carbonat, Methylsalicylat-phenyl-carbonat, Di-(methylsalicylat)-carbonat, Ethylsalicylat-phenyl-carbonat, Di-(ethylsalicylat)-carbonat, n-Propylsalicylat-phenyl-carbonat, Di-(n-propylsalicylat)-carbonat, iso-Propylsalicylat-phenyl-carbonat, Di-(iso-propylsalicylat)-carbonat, n-Butylsalicylat-phenyl-carbonat, Di-(n-butylsalicylat)-carbonat, iso-Butylsalicylat-phenyl-carbonat, Di-(iso-butylsalicylat)-carbonat, tert-Butylsalicylat-phenyl-carbonat, Di-(tert-butylsalicylat)-carbonat, Di-(phenylsalicylat)-carbonat und Di-(benzylsalicylat)-carbonat.

Bevorzugte Diarylcarbonate sind: Diphenylcarbonat, 4-tert-Butylphenyl-phenyl-carbonat, Di-(4-tert-butylphenyl)-carbonat, Biphenyl-4-yl-phenyl-carbonat, Di-(biphenyl-4-yl)-carbonat, 4-(1-Methyl-1-phenylethyl)-phenyl-phenyl-carbonat und Di-[4-(1-methyl-1-phenylethyl)-phenyl]-carbonat.

Besonders bevorzugt ist Diphenylcarbonat.

Im Rahmen der Erfindung bevorzugt eingesetzte Dialkylcarbonate sind solche der Formel (II) 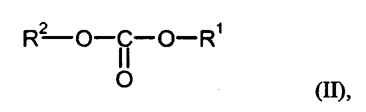 wobei R¹ und R² unabhängig voneinander für lineares oder verzweigtes, gegebenenfalls substituiertes C₁-C₃₄-Alkyl, bevorzugt für C₁-C₆-Alkyl, besonders bevorzugt für C₁-C₄-Alkyl stehen. Dabei können R¹ und R² gleich oder verschieden sein. Bevorzugt sind R¹ und R² gleich.

Bevorzugte Dialkylcarbonate sind Dimethylcarbonat, Diethylcarbonat, Di(n-propyl)carbonat, Di(iso-propyl)carbonat, Di(n-butyl)carbonat, Di(sec-butyl)carbonat, Di(tert-butyl)carbonat oder Dihexylcarbonat. Besonders bevorzugt sind Dimethylcarbonat oder Diethylcarbonat. Ganz besonders bevorzugt ist Dimethylcarbonat.

Im Rahmen der Erfmdung geeignete aromatische Hydroxyverbindungen sind bevorzugt solche der allgemeinen Formel (III) 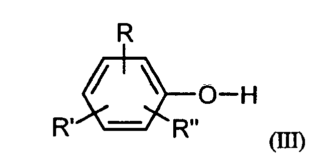 worin R, R' und R" unabhängig voneinander die für die allgemeine Formel (I) genannte Bedeutung haben können.

Solche aromatischen Hydroxyverbindungen sind beispielsweise: Phenol, o-, m- oder p-Kresol, auch als Gemisch der Kresole, Dimethylphenol, auch als Gemisch, wobei die Stellung der Methylgruppen am Phenolring beliebig sein kann, z.B. 2,4-, 2,6-, oder 3,4-Dimethylphenol, o-, moder p-Chlorphenol, o-, m- oder p-Ethylphenol, o-, m- oder p-n-Propylphenol, 4-iso-Propylphenol, 4-n-Butylphenol, 4-iso-Butylphenol, 4-tert-Butylphenol, 4-n-Pentylphenol, 4-n-Hexylphenol, 4-isoOctylphenol, 4-n-Nonylphenol, o-, m- oder p-Methoxyphenol, 4-Cyclohexylphenol, 4-(1-Methyl-1-phenylethyl)-phenol, Biphenyl-4-ol, 1-Naphthol, 2-1-Naphthol, 4-(1-Naphthyl)phenol, 4-(2-Naphthyl)-phenol, 4-Phenoxyphenol, 3-Pentadecylphenol, 4-Tritylphenol, Methylsalicylsäure, Ethylsalicylsäure, n-Propylsalicylsäuret, iso-Propylsalicylsäure, n-Butylsalicylsäure, iso-Butylsalicylsäure, tert-Butylsalicylsäure, Phenylsalicylsäure und Benzylsalicylsäure.

Bevorzugte aromatischen Hydroxyverbindungen sind Phenol, 4-tert-Butylphenol, Biphenyl-4-ol und 4-(1-Methyl-1-phenylethyl)-phenol.

Besonders bevorzugt ist Phenol.

Im Rahmen der Erfindung hergestellte Alkylarylcarbonate sind bevorzugt solche der allgemeinen Formel (IV) 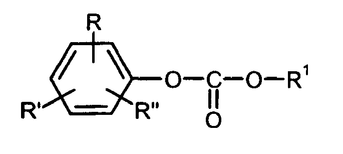 worin R, R' und R" die für die allgemeine Formel (I) und R¹ die für die allgemeine Formel (II) genannte Bedeutung haben können.

Bevorzugte Alkylarylcarbonate sind Methyl-phenyl-carbonat, Ethyl-phenyl-carbonat, Propyl-phenyl-carbonat, Butylphenyl-carbonat und Hexyl-phenyl-carbonat, Methyl-(o-kresyl)-carbonat, Methyl-(p-kresyl)-carbonat, Ethyl-(o-kresyl)-carbonat, Ethyl-(p-kresyl)-carbonat, Methyl- oder Ethyl-(p-chlorphenyl)-carbonat. Besonders bevorzugte Alkylarylcarbonate sind Methyl-phenyl-carbonat und Ethyl-phenyl-carbonat. Ganz besonders bevorzugt ist Methyl-phenyl-carbonat.

Sowohl die für das erfmdungsgemäße Verfahren geeigneten Dialkylcarbonate als auch die aromatischen Hydroxyverbindungen sind dem Fachmann bekannt und kommerziell erhältlich oder können nach dem Fachmann ebenfalls bekannten Verfahren hergestellt werden.

**C₁-C₄-Alkyl** steht im Rahmen der Erfmdung beispielsweise für Methyl, Ethyl, n-Propyl, iso-Propyl, n-Butyl, sec.-Butyl, tert.-Butyl, **C₁-C₆-Alkyl** darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, **C₁-C₃₄-Alkyl** darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

**Aryl** steht für einen carbocyclischen aromatischen Rest mit 6 bis 34 Gerüstkohlenstoffatomen. Gleiches gilt für den aromatischen Teil eines Arylalkylrestes, auch Aralkylrest genannt, sowie für Arylbestandteile komplexerer Gruppen, wie z.B. Arylcarbonylresten.

**Arylalkyl** bzw. **Aralkyl** bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Defmition substituiert sein kann.

Die vorangehenden Aufzählungen sind beispielhaft und nicht als Limitierung zu verstehen.

Im erfindungsgemäßen Verfahren werden die aromatische(n) Hydroxyverbindung(en) und das oder die Dialkylcarbonat(e) bevorzugt im Molverhältnis von 1 : 0.1 bis 1 : 10, besonders bevorzugt von 1 : 0.2 bis 1 : 5, ganz besonders bevorzugt von 1 : 0.5 bis 1 : 3 in der ersten Reaktionskolonne eingesetzt. Dabei berücksichtigt das angegebene Molverhältnis nicht die Rückführung von aromatischer Hydroxyverbindung oder Dialkylcarbonat in die Reaktionskolonne über einen oder mehrere Kopfkondensator(en) (vgl. unter (b)) oder einen oder mehrere etwaig vorhandene(n) Sumpfverdampfer.

Das erfindungsgemäße Verfahren wird in mindestens zwei Reaktionskolonnen durchgeführt.

Als erste und zweite Reaktionskolonne oder gegebenenfalls dritte bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Die erste Reaktionskolonne enthält wenigstens einen Verstärkungsteil im oberen Teil der Kolonne und wenigstens eine Reaktionszone unterhalb des Verstärkungsteils, welcher mindestens zwei Sektionen aufweist. Jede der zwei Sektionen weist unabhängig voneinander bevorzugt jeweils 0 bis 20, bevorzugt 0,1 bis 20 theoretische Stufen auf. In bevorzugten Ausführungsformen ist wenigstens ein Verstärkungsteil der ersten Reaktionskolonne mit wenigstens einem Zwischenkondensator ausgestattet. Der Zwischenkondensator ist vorzugsweise zwischen den beiden Sektionen des Verstärkungsteils angebracht. In diesem Fall wird der Verstärkungsteil in einen oberen und einen unteren Verstärkungsteil geteilt.

Die erste Reaktionskolonne wird bevorzugt im Gegenstrom betrieben, wobei vorzugsweise in wenigstens einer Reaktionszone dieser Kolonne die aromatische Hydroxyverbindung flüssig vom Kopf zum Sumpf geführt und das Dialkylcarbonat gasförmig diesem flüssigen Strom entgegengeführt wird. Dabei wird die erste Reaktionskolonne vorzugsweise so betrieben, dass man wenigstens einer Reaktionszone, bevorzugt in das obere Drittel der Reaktionszone, einen oder mehrere, die aromatische Hydroxyverbindung und gegebenenfalls gelösten Umesterungskatalysator enthaltende Ströme, bevorzugt mit der an dieser Stelle der Kolonne herrschenden Temperatur, flüssig oder mit nur geringem Gasanteil eindosiert, wobei der Gasanteil bevorzugt weniger als 20 Gew.-% beträgt. Zudem werden einer oder mehrere, das Dialkylcarbonat enthaltende Ströme in die Reaktionszone, bevorzugt im unteren Drittel dieser Reaktionszone, gleitet, wobei die Zudosierung vorzugsweise gasförmig oder überhitzt erfolgt. In bevorzugten Ausführungsformen kann die Überhitzung des Dampfstroms 0 bis 50 °C betragen. Des Weiteren richtet sich die Taupunktstemperatur bevorzugt nach dem in der Reaktionszone an der Zudosierstelle des jeweiligen Dialkylcarbonat enthaltenden Stromes vorliegenden Druck.

Nach Passieren der Reaktionszone(n) wird der während der Reaktion gebildete Alkylalkohol, nach Durchlaufen des oder der Verstärkungsteile, am Kopf der ersten Reaktionskolonne entnommen. Beim während der Reaktion gebildeten Alkylalkohol, auch Reaktionsalkohol genannt, handelt es sich im Rahmen der Erfmdung um den bei der Umesterung frei werdenden Alkohol, bevorzugt R'-OH bzw. R²-OH, wobei R¹ und R² die für die allgemeine Formel (II) genannte Bedeutung haben. Der am Kopf der ersten Reaktionskolonne entnommene Strom enthält im Allgemeinen zusätzlich zum während der Reaktion gebildeten Alkylalkohol noch überschüssiges oder nicht umgesetztes Dialkylcarbonat und leichtsiedende Nebenverbindungen, wie beispielsweise Kohlendioxid oder Dialkylether. Aufgrund des oder der vorhandenen Verstärkungsteil(e) enthält dieser Strom nur geringe Mengen an höher siedenden Komponenten, wie z.B. der aromatischen Hydroxyverbindung. Der Verstärkungsteil dient zur Abtrennung der in der Reaktionszone mitverdampften höher siedenden Komponenten, wie z.B. der aromatischen Hydroxyverbindung oder Alkylarylcarbonat von den leichtsiedenden Reaktionsalkoholen oder Dialkylcarbonaten. Dies hat den Vorteil, dass die Trennung der während der Reaktion gebildeten Alkylalkohole von den Dialkylcarbonaten bei einem niedrigen Temperaturniveau durchgeführt werden kann.

Die erste Reaktionskolonne wird in bevorzugten Ausführungsformen unter Rückflussbedingungen betrieben. Unter Rückflussbedingungen ist eine solche Fahrweise zu verstehen, bei der man den Dampfstrom am oberen Ende des Verstärkungsteils teilweise oder vollständig kondensiert (vgl. unter (b)) und das dabei anfallende Kondensat teilweise oder vollständig wieder als Rücklauf auf das obere Ende des Verstärkungsteils zurückführt. Das Rücklaufverhältnis beträgt dabei bevorzugt 0,1 bis 20, besonders bevorzugt 0,1 bis 10 und ganz besonders bevorzugt 0,1 bis 3, wobei das Rücklaufverhältnis im Rahmen der Erfindung dem Gewichtsverhältnis von in die Kolonne zurückgeführtem Kondensat zu am Kopf der Kolonne entnommenem Dampf ohne zurückgeführtes Kondensat entspricht.

In bevorzugten Ausführungsformen weist die erste Reaktionskolonne unterhalb einer Reaktionszone wenigstens einen Abtriebsteil auf.

Die erste Reaktionskolonne kann weiterhin bevorzugt mit einem oder mehreren Sumpfverdampfer(n) ausgestattet sein. Bei Ausführung der ersten Reaktionskolonne mit einem Abtriebsteil wird bevorzugt zusätzlich ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die erste Reaktionskolonne zurückgeführt. Im Falle einer Ausführungsform ohne Abtriebsteil wird in einem gegebenenfalls eingesetzten Sumpfverdampfer die aus der Reaktionszone ablaufende Flüssigkeit ganz oder teilweise verdampft und ganz oder teilweise wieder in die erste Reaktionskolonne zurückgeführt.

In den bevorzugten Ausführungsformen, in denen wenigstens ein Verstärkungsteil der ersten Reaktionskolonne mit wenigstens einem Zwischenkondensator ausgestattet ist, ist der Verstärkungsteil der ersten Reaktionskolonne, der mit wenigstens einem Zwischenkondensator ausgestattet ist, in einen unteren und einen oberen Verstärkungsteil (zwei Sektionen) unterteilt, wovon sich der untere Verstärkungsteil unterhalb des Zwischenkondensators und der obere Verstärkungsteil oberhalb des Zwischenkondensators befinden.

Der oder die Verstärkungsteil(e) mit wenigstens einem Zwischenkondensator können in bevorzugten Ausführungsformen zusammen mit dem oder den Reaktionsteil(en) und gegebenenfalls wenigstens einem Abtriebsteil in der Reaktionskolonne untergebracht werden. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung der aromatischen Hydroxyverbindung stattfindet. Das aus dieser unteren Sektion bzw. gegebenenfalls dem unteren Verstärkungsteil kommende dampfförmige Gemisch wird in einen Zwischenkondensator geleitet, wo dieses teilweise auskondensiert und das anfallende Kondensat am oberen Ende der unteren Sektion des Verstärkungsteils bzw. gegebenenfalls dem unteren Verstärkungsteil zugeführt wird.

In einer weiteren bevorzugten Ausführungsform des erfindungsgemäßen Verfahrens wird der Zwischenkondensator nicht in die erste Reaktionskolonne integriert sondern als separater Zwischenkondensator außerhalb der ersten Reaktionskolonne ausgeführt.

In einer weiteren bevorzugten Ausführungsform des erfindungsgemäßen Verfahrens werden Zwischenkondensator und die obere Sektion des Verstärkungsteils nicht in die Reaktionskolonne integriert sondern separat außerhalb der ersten Reaktionskolonne untergebracht.

Unterhalb der Reaktionszone und eines gegebenenfalls vorhandenen Abtriebsteils wird ein Gemisch enthaltend Alkylarylcarbonat, überschüssigem oder nichtumgesetztem Phenol, Diarylcarbonat, Umesterungskatalysatoren, Dialkylcarbonat, Reaktionsalkohol und bei der Reaktion entstehende oder bereits in den Edukten enthaltene schwersiedende Verbindungen erhalten. Bei Verwendung eines Abtriebsteils wird der Gehalt an leichtsiedenden Verbindungen, wie beispielsweise Dialkylcarbonat und Reaktionsalkohol reduziert, wobei in Anwesenheit des Umesterungskatalysators unter Umständen weiteres Alkylarylcarbonat und/oder Diarylcarbonat gebildet werden. Die hierzu erforderliche Energie, wird bevorzugt durch einen oder mehrere Verdampfer zugeführt.

In allen Abschnitten der ersten Reaktionskolonne, d.h. sowohl in Verstärkungs- und gegebenenfalls Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl,, Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novaloxringe, Berlsättel, BX-Packungen, Montz Pak, Mellapak, Melladur, Kerapak und CY-Packungen.

Alternativ eignen sich auch Kolonnenböden, wie beispielesweise Sieb-, Glocken-, Ventil-, Tunnelböden. In der oder den Reaktionszone(n) der Reaktionskolonne sind Kolonnenböden mit hohen Verweilzeiten bei gutem Stoffaustausch, beispielsweise Glockenböden, Ventil- oder Tunnelböden mit hohen Überlaufwehren, besonders bevorzugt. Die theoretische Bodenzahl der Reaktionszone beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 100 bis 300°C, besonders bevorzugt von 120 bis 250°C, ganz besonders bevorzugt von 150 bis 240°C. In bevorzugten Ausführungsformen wird in der Reaktionszone eine optimale Reaktionstemperatur einerseits durch die Wahl der Betriebsbedingungen und andererseits durch zusätzliche Wärmzufuhr im Bereich eines oder mehrerer Reaktionsböden eingestellt. Die Wärmezufuhr auf den Reaktionsböden kann dabei entweder durch Wärmetauscher oder über Reaktionsböden mit Möglichkeit zum Wärmeeintrag erfolgen. Es ist von Vorteil, die erfindungsgemäße Umesterung nicht nur bei Normaldruck, sondern auch bei erhöhtem oder erniedrigtem Druck durchzuführen. Der Druck der Reaktionszone liegt daher bevorzugt im Bereich von 0.5 bis 20 bar (absolut), besonders bevorzugt von 0.8 bis 15 bar (absolut), ganz besonders bevorzugt von 0.9 bis 10 bar (absolut).

Für die in der ersten Reaktionskolonne auftretenden Reaktionsschritte können aus der Literatur bekannte Umesterungskatalysatoren eingesetzt werden. Dies sind aus der Literatur bekannte Umesterungskatalysatoren für die Dialkylcarbonat-Phenol-Umesterung, wie z. B. Hydride, Oxide, Hydroxide, Alkoholate, Amide und andere Salze von Alkali- und Erdalkalimetallen, wie von Lithium, Natrium, Kalium, Rubidium, Caesium, Magnesium und Calcium, bevorzugt Lithium, Natrium, Kalium, Magnesium und Calcium und besonders bevorzugt Lithium, Natrium und Kalium (vgl. z.B. US 3 642 858, US 3 803 201 oder EP-A 1082). Salze der Alkali- und Erdalkalimetalle können auch solche von organischen oder anorganischen Säuren sein, wie von Essigsäure, Propionsäure, Buttersäure, Benzoesäure, Stearinsäure, Kohlensäure (Carbonate oder Hydrogencarbonate), Phosphorsäure, Blausäure, Rhodanwasserstoffsäure, Borsäure, Zinnsäure, C₁₄-Stannonsäuren oder Antimonsäure. Bevorzugt kommen als Verbindungen der Alkali- und Erdalkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt. Die genannten Alkali- oder Erdalkalimetallverbindungen werden bevorzugt in Mengen von 0,001 bis 2 Gew.-%, bevorzugt von 0,005 bis 0,9 Gew.-% und besonders bevorzugt von 0,01 bis 0,5 Gew.-%, bezogen auf das Gewicht des umzusetzenden Reaktionsgemischs, eingesetzt.

Weitere erfindungsgemäß einsetzbare Katalysatoren sind Metallverbindungen wie AlX₃, TiX₃, UX₄, TiX₄, VOX₃, VX₅, ZnX₂, FeX₃, PbX₂ und SnX₄, worin X für Halogen-, Acetoxy-, Alkoxy- oder Aryloxyreste steht (DE-OS 2 58 412). Besonders bevorzugte erfindungsgemäß einsetzbare Katalysatoren sind Metallverbindungen wie AlX₃, TiX₄, PbX₂ und SnX₄, wie beispielsweise Titantetrachlorid, Titantetramethoxid Titantetraphenoxid, Titantetraethoxid, Titantetraisopropylat, Titantetradodecylat, Zinntetraisooctylat und Aluminiumtriisopropylat. Ganz besonders bevorzugt sind Metallverbindungen TiX₄. Die genannten Metallverbindungen werden bevorzugt in Mengen von 0,001 bis 5 Gew.-%, bevorzugt von 0,005 bis 5 Gew.-% und besonders bevorzugt von 0,01 bis 5 Gew.-%, bezogen auf das Gewicht des umzusetzenden Reaktionsgemischs, eingesetzt.

Halogen bedeutet im Rahmen der Erfmdung Fluor, Chlor oder Brom, bevorzugt Fluor oder Chlor, besonders bevorzugt Chlor.

Weitere erfindungsgemäß einsetzbare Katalysatoren sind zinnorganische Verbindungen der allgemeinen Formel (R¹¹)_{4-X}-Sn(Y)_{X}, in der Y für einen Rest OCOR¹², OH oder OR steht, wobei R¹² C₁-C₁₂-Alkyl, C₆-C₁₂-Aryl oder C₇-C₁₃-Alkylaryl bedeutet, R¹¹ unabhängig von R¹² die Bedeutung von R¹² hat und x eine ganze Zahl von 1 bis 3 bedeutet, Dialkylzinnverbindungen mit 1 bis 12 C-Atomen im Alkylrest oder Bis-(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew: % (vgl. EP 879, EP 880, EP 39 452, DE-OS 34 45 555, JP 79/63023), polymere Zinnverbindungen der Formel -[-RR¹¹Sn-O-]-, in welcher R und R¹¹ unabhängig voneinander die vorangehend für R¹² genannte Bedeutung haben, beispielsweise Poly[oxy(dibutylstannylen)] Poly[oxy(dioctylstannylen)], Poly[oxy(butylphenylstannylen)] und Poly[oxy(diphenylstannylen)] (DE-OS 34 45 552), polymere Hydroxystannoxane der Formel -[-RSn(OH)-O-]-, beispielsweise Poly(ethylhydroxystannoxan), Poly(butylhydroxystannoxan), Poly-(octylhydroxystannoxan), Poly(undecylhydroxystannoxan) und Poly(dodecylhydroxystannoxane) in Mengen von 0,001 bis 20 Gew.-%, bevorzugt von 0,005 bis 5 Gew.-%, bezogen auf Dialkylcarbonat (DE-OS 40 06 520). Weitere erfindungsgemäß einsetzbare Zinnverbindungen sind Sn(II)oxide der allgemeinen Formel

X-R₂Sn-O-R₂Sn-Y,

worin X und Y unabhängig voneinander OH, SCN, OR¹³, OCOR¹³ oder Halogen und R Alkyl, Aryl bedeuten soll, worin R¹³ die vorangehend für R¹² genannte Bedeutung hat (EP 0 338 760).

Als weitere erfindungsgemäß einsetzbare Katalysatoren kommen Bleiverbindungen, gegebenenfalls zusammen mit Triorganophosphanen, einer Chelatverbindung oder einem Alkalimetallhalogenid, beispielsweise Pb(OH)₂-2PbCO₃, Pb(OCO-CH₃)₂, Pb(OCO-CH₃)₂ ·2LiCl, Pb(OCO-CH₃)₂ • 2PPh₃ in Mengen von 0,001 bis 1, bevorzugt von 0,005 bis 0,25 Mol pro Mol Dialkylcarbonat (JP 57/176932, JP 01/093580), sowie andere Blei(II)- und Blei(IV)-verbindungen, wie PbO, PbO₂, Mennige, Plumbite, und Plumbate (JP 01/093560), Eisen(III)acetat (JP 61/1 72 852), weiterhin Kupfersalze und/oder Metallkomplexe, beispielsweise von Alkali, Zink, Titan und Eisen (JP 89/005588).

Weiterhin sind in den erfmdungsgemäßen Verfahren heterogene Katalysatorsysteme einsetzbar. Solche sind beispielsweise Mischoxide aus Silizium und Titan, die durch gemeinsame Hydrolyse von Silizium und Titanhalogeniden erhältlich sind (JP 54/125617) oder Titandioxide mit hoher BET-Oberfläche >20 m²/g (DE-OS 40 36 594)).

Bevorzugte Katalysatoren für das erfindungsgemäße Verfahren sind die vorangehend genannten Metallverbindungen AlX₃, TiX₃, UX₄, TiX₄, VOX₃, VX₅, ZnX₂, FeX₃, PbX₂ und SnX₄. Besonders bevorzugt sind AlX₃, TiX₄, PbX₂ und SnX₄, wovon beispielhaft Titantetrachlorid, Titantetramethoxid Titantetraphenoxid, Titantetraethoxid, Titantetraisopropylat, Titantetradodecylat, Zinntetraisooctylat und Aluminiumtriisopropylat genannt seien. Ganz besonders bevorzugt sind Metallverbindungen TiX₄. Insbesondere bevorzugt sind Titantetramethoxid, Titantetraphenoxid und Titantetraethoxid.

Der Katalysator wird bevorzugt zusammen mit dem Strom enthaltend die aromatische(n) Hydroxyverbindung(en) in gelöster oder suspendierter Form in die erste Reaktionskolonne eingebracht. Alternativ kann der Katalysator auch separat beispielsweise in einem dem Reaktionsalkohol entsprechenden Alkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Die für die Reaktion in der ersten Reaktionskolonne erforderliche Energie kann einerseits über innen oder außen liegende Vorrichtungen, wie z.B. Wärmetauscher, Verdampfer und/oder beheizbare Kolonnenböden, erzeugt und/oder andererseits sowohl mit dem flüssigen Strom enthaltend die aromatische(n) Hydroxyverbindung(en) als auch mit dem gasförmig eindosierten, Dialkylcarbonat enthaltenden Strom eingebracht werden. Insbesondere im Bereich der Reaktionszone(n) kann eine Zufuhr von Wärme auf diese Weise erfolgen. Vorzugsweise wird diese Wärme im Bereich der Reaktionszone(n) ganz oder teilweise mittels Verdampfer oder beheizbarer Kolonnenböden zugeführt. Besonders vorteilhaft ist es, die für die Reaktion in der ersten Reaktionskolonne erforderliche Energie wenigstens teilweise sowohl mit dem flüssigen Strom enthaltend die aromatische(n) Hydroxyverbindung(en) als auch mit dem gasförmig eindosierten, Dialkylcarbonat enthaltenden Strom in die ersten Reaktionskolonne und zusätzlich durch innen- und/oder außen liegende Wärmetauscher einzubringen.

Im erfmdungsgemäßen Verfahren wird das Sumpfprodukt der ersten Reaktionskolonne einer zweiten Reaktionskolonne zugeführt.

Die zweite Reaktionskolonne enthält wenigstens einen Verstärkungsteil im oberen Teil der Kolonne und wenigstens eine Reaktionszone unterhalb des Verstärkungsteils. Der Verstärkungsteil weist bevorzugt 1 bis 50, besonders bevorzugt 1 bis 25 theoretische Stufen auf.

In der zweiten Reaktionskolonne wird das Sumpfpropdukt der ersten Reaktionskolonne, welches bereits gebildetes Alkylarylcarbonat und Diarylcarbonat enthält, flüssig oder als Dampf-Flüssigkeits-Gemisch bevorzugt der Reaktionszone, besonders bevorzugt dem oberen Teil der Reaktionszone, ganz besonders bevorzugt im oberen Drittel der Reaktionszone zugeführt. Dabei wird die zweite Reaktionskolonne vorzugsweise so betrieben, dass man das Alkylarylcarbonat teilweise oder vollständig, beispielsweise durch weitere Umesterung oder Disproportionierung, bevorzugt durch Disproportionierung, zum Diarylcarbonat umsetzt. Zusätzlich zum Sumpfpdrodukt der ersten Reaktionskolonne können einer oder mehrere, Alkylarylcarbonat enthaltende Ströme flüssig oder als Dampf-Flüssigkeitsgemisch im Bereich der Reaktionszone eindosiert werden. Solche zusätzlichen Alkylarylcarbonat enthaltenden Ströme können beispielsweise aus der weiteren Aufarbeitung stammen und so in das Verfahren zurückgeführt werden.

Am Kopf der zweiten Reaktionskolonne werden nicht umgesetzte aromatische Hydroxyverbindung, Dialkylcarbonat, Reaktionsalkohol, mittelsiedende Nebenverbindungen - wie beispielsweise Alkylarylether - und in geringem Maße leichtsiedende Nebenverbindungen abgetrennt. Im Rahmen der Erfmdung sind unter mittelsiedenden Nebenverbindungen solche mit einem Siedepunkt unterhalb dem der aromatischen Hydroxyverbindung und oberhalb dem des Dialkylcarbonats zu verstehen. Solche mittelsiedenden Nebenverbindungen sind z.B. Alkylarylether, wie beispielsweise Anisol oder Phenetol. Die in der zweiten Reaktionskolonne abgetrennten mittelsiedenden Nebenverbindungen können in der ersten und/oder zweiten Reaktionskolonne bei der Reaktion entstehen oder bereits durch die Edukte in das Verfahren eingeschleust worden sein.

Der Verstärkungsteil der zweiten Reaktionskolonne dient zur Abtrennung der in der Reaktionszone mitverdampften höher siedenden Komponenten, wie z.B. Alkylarylcarbonat.

Die zweite Reaktionskolonne wird in bevorzugten Ausführungsformen unter Rückflussbedingungen betrieben. Unter Rückflussbedingungen ist eine solche Fahrweise zu verstehen, bei der man den Dampfstrom am oberen Ende des Verstärkungsteils teilweise oder vollständig kondensiert und das dabei anfallende Kondensat teilweise oder vollständig wieder als Rücklauf auf das obere Ende des Verstärkungsteils zurückführt. Das Rücklaufverhältnis beträgt dabei bevorzugt 0,1 bis 20, besonders bevorzugt 0,1 bis 10 und ganz besonders bevorzugt 0,1 bis 3, wobei das Rücklaufverhältnis im Rahmen der Erfmdung dem Gewichtsverhältnis von in die Kolonne zurückgeführtem Kondensat zu am Kopf der Kolonne entnommenem Dampf ohne zurückgeführtes Kondensat entspricht.

Die zweite Reaktionskolonne kann unterhalb einer Reaktionszone wenigstens einen Abtriebsteil aufweisen. In bevorzugten Ausführungsformen kann die Reaktionszone der zweiten Reaktionskolonne jedoch gleichzeitig als Abtriebsteil fungieren. Dabei wird das bei der Disproportionierung freigesetzte Dialkylcarbonat, durch Umesterung freigesetzter Reaktionsalkohol und nicht umgesetzte aromatische Hydroxyverbindung abgetrennt und gleichzeitig Diarylcarbonat und das im Wesentlichen durch Disproportionierung abreagierende Alkylarylcarbonat aufkonzentriert.

Die zweite Reaktionskolonne kann weiterhin bevorzugt mit einem oder mehreren Sumpfverdampfer(n) ausgestattet sein.

Prinzipiell kann der Verstärkungsteil der zweiten Reaktionskolonne ebenfalls mit einem oder mehreren Zwischenkondensatoren ausgestattet werden. Hierdurch wird der Verstärkungsteil, in einen unteren und einen oberen Verstärkungsteil (zwei Sektionen) unterteilt, wovon sich der untere Verstärkungsteil unterhalb des Zwischenkondensators und der obere Verstärkungsteil oberhalb des Zwischenkondensators befinden. In einer bevorzugten Ausführungsform weist wird die zweite Reaktionskolonne keinen Zwischenkondensator auf.

Die zweite Reaktionskolonne ist mit einem oder mehreren Kondensatoren ausgestattet. Bevorzugt handelt es sich dabei um einen oder mehrere Kondensatoren am Kopf der zweiten Reaktionskolonne (Kopfkondensator(en)). Besonders bevorzugt wird eine Kaskade von Kopfkondensatoren verwendet.

Im Laufe der Kondensation in dem oder den Kondensator(en) am Kopf der zweiten Reaktionskolonne verarmen die Dämpfe an höher siedenden Komponenten, wie z.B. aromaticher Hydroxyverbindung. Um die anfallende Kondensationswärme im Sinne einer Wärmeintegration besonders effizient nutzen zu können, erfolgt die Kondensation daher bevorzugt mehrstufig, besonders bevorzugt mindestens zweistufig, in bevorzugten Ausführungsformen zwei- oder dreistufig.

Bei der besonders bevorzugten Ausführungsform der zwei- oder dreistufigen Kondensation wird die Kondensationswärme der ersten oder der ersten und zweiten Kondensationsstufe direkt oder indirekt zum Erwärmen eines Stoffstromes oder einer Kolonne innerhalb des Verfahrens verwendet, während die anfallende Kondensationswärme der zweiten oder dritten Kondensationsstufe durch Kühlwasser oder Luftkühlung abgeführt wird.

Die Kondensation am Kopf der zweiten Reaktionskolonne kann in weiteren bevorzugten Ausführungsformen zudem so durchgeführt werden, dass ein Teil der am Kopf der zweiten Reaktionskolonne entnommenen Dämpfe nicht kondensiert wird, um mittelsiedende Nebenverbindungen selektiv ausschleusen zu können.

Unterhalb der Reaktionszone und eines gegebenenfalls vorhandenen Abtriebsteils wird ein Gemisch enthaltend Alkylarylcarbonat, überschüssiger oder nichtumgesetzter aromatischer Hydroxyverbindung, Diarylcarbonat, Umesterungskatalysator(en), Dialkylcarbonat, Reaktionsalkohol und bei der Reaktion entstehende oder bereits in den Edukten enthaltene mittel-oder schwersiedende Nebenverbindungen erhalten. Im Rahmen der Erfindung sind unter schwersiedenden Nebenverbindungen solche mit einem Siedepunkt oberhalb dem der aromatischen Hydroxyverbindung zu verstehen.

In allen Abschnitten der zweiten Reaktionskolonne, d.h. sowohl in Verstärkungs- und gegebenenfalls Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl,, Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novolaxringe, Berlsättel, BX-Packungen, Montz Pak, Mellapak, Melladur, Kerapak und CY-Packungen.

Alternativ eignen sich auch Kolonnenböden, wie beispielesweise Sieb-, Glocken-, Ventil-, Tunnelböden. In der oder den Reaktionszone(n) der zweiten Reaktionskolonne sind Füllkörperschüttungen oder strukturierte Packungen besonders bevorzugt. Die theoretische Bodenzahl der Reaktionszone beträgt vorzugsweise 3 bis 50, besonders bevorzugt 10 bis 50 und ganz besonders bevorzugt 10 bis 40.

Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 100 bis 300°C, besonders bevorzugt von 120 bis 250°C, ganz besonders bevorzugt von 180 bis 250°C.

In besonderen Ausführungsformen wird in der Reaktionszone eine optimale Reaktionstemperatur einerseits durch die Wahl der Betriebsbedingungen und andererseits durch zusätzliche Wärmzufuhr im Bereich eines oder mehrerer Reaktionsböden eingestellt. Die Wärmezufuhr auf den Reaktionsböden kann dabei entweder durch Wärmetauscher oder über Reaktionsböden mit Möglichkeit zum Wärmeeintrag erfolgen. Es ist von Vorteil, die erfindungsgemäße Umesterung nicht nur bei Normaldruck, sondern auch bei erhöhtem oder erniedrigtem Druck, bevorzugt bei erniedrigtem Druck, durchzuführen. Der Druck der zweiten Reaktionskolonne liegt daher bevorzugt im Bereich von 0.05 bis 20 bar (absolut), besonders bevorzugt von 0.1 bis 10 bar (absolut), ganz besonders bevorzugt von 0.1 bis 2 bar (absolut).

Für die in der zweiten Reaktionskolonne auftretenden Reaktionsschritte können die vorangehend bereits für die Umesterung in der ersten Reaktionskolonne genannten Umesterungskatalysatoren eingesetzt werden. In einer bevorzugten Ausführungsform werden in der ersten und zweiten Reaktionskolonne identische Katalysatoren verwendet.

Der Katalysator wird bevorzugt zusammen mit dem Sumpfprodukt der ersten Reaktionskolonne in gelöster oder suspendierter Form in die zweite Reaktionskolonne eingebracht. Alternativ kann der Katalysator auch separat beispielsweise in einem dem Reaktionsalkohol entsprechenden Alkohol oder einem geeigneten inerten Lösungsmittel zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Die für die Reaktion in der zweiten Reaktionskolonne erforderliche Energie kann einerseits über innen oder außen liegende Vorrichtungen, wie z.B. Wärmetauscher, Verdampfer und/oder beheizbare Kolonnenböden, erzeugt und/oder andererseits sowohl mit dem flüssigen Strom enthaltend die aromatische(n) Hydroxyverbindung(en) eingebracht werden. Vorzugsweise wird diese Wärme im Bereich der Reaktionszone(n) ganz oder teilweise mittels Verdampfer zugeführt.

An die zweite Reaktionskolonne können sich eine oder mehrere weitere Reaktionskolonnen anschließen. Für solche weiteren Reaktionskolonnen gelten die vorangehend für die zweite Reaktionskolonne genannten Bedingungen und Parameterbereiche, wobei die Bedingungen und Parameter weiterer Reaktionskolonnen jedoch nicht identisch zu denen in der zweiten Reaktionskolonne sein müssen, sondern sich bevorzugt innerhalb des vorangehend genannten Rahmens der Bedingungen und Parameterbereiche von denen in der zweiten Reaktionskolonne unterscheiden. Vorzugsweise wird eine zur zweiten Reaktionskolonne zusätzliche Reaktionskolonne beispielsweise bei niedrigerem Druck betrieben als die zweite Reaktionskolonne; auch Rücklaufverhältnis und Sumpftemperatur können gegenüber denen in der zweiten Reaktionskolonne verändert sein. In einer bevorzugten Ausführungsform schließt sich im erfindungsgemäßen Verfahren an die erste Reaktionskolonne lediglich eine weitere Reaktionskolonne, d.h. die vorangehend genannte zweite Reaktionskolonne an. An die Reaktionskolonnen können sich jedoch weitere Kolonnen zur Aufreinigung und Trennung der Komponenten der entnommenen Ströme anschließen. Solche Kolonnen zur Aufreinigung und Trennung der Komponenten werden im Rahmen der Erfmdung nicht als Reaktionskolonnen im Sinne der Erfindung verstanden.

Die durch Kondensation in dem oder den Kondensator(en), bevorzugt Kopfkondensator(en) der zweiten oder den weiteren Reaktionskolonne(n), vorzugsweise der zweiten Reaktionskolonne, gewonnene Kondensationswärme wird erfindungsgemäß direkt oder indirekt, ganz oder teilweise wieder in das Verfahren zurückgeführt. Unter direkter Rückführung der Kondensationswärme in das Verfahren ist im Rahmen der Erfindung zu verstehen, dass diese Kondensationswärme ohne zwischengeschaltetes Heizmedium in das Verfahren zurückgeführt wird, z.B. zur Erwärmung entweder eines oder mehrerer Ströme oder zur Erwärmung eines oder mehrerer Kolonnenabschnitte innerhalb des Verfahrens. Dies kann beispielsweise in einem Wärmetauscher erfolgen. Vorzugsweise ist dabei entweder ein solcher Wärmeaustauscher mit dem oder den Kondensatoren kombiniert. Unter indirekter Rückführung der Kondensationswärme in das Verfahren ist im Rahmen der Erfindung zu verstehen, dass mit der gewonnen Kondensationswärme zunächst ein Heizmedium erzeugt wird, welches zur Rückführung der Kondensationswärme in das Verfahren dient. Mit diesem Heizmedium können beispielsweise ein oder mehrere Ströme oder einer oder mehrere Kolonnenabschnitte innerhalb des Verfahrens erwärmt werden. Als Heizmedium kommen Gase, Dämpfe oder Flüssigkeiten in Frage, bevorzugt dampfförmige oder flüssige technische Wärmeträgermedien wie beispielsweise Wasser, Wärmeträger auf Mineralölbasis oder synthetische Wärmeträger (z.B. Diphyl^{™}, Marlotherm^{®}). Besonders bevorzugte Heizmedien sind Wasser oder Wasserdampf.

Für den Fall, dass die erste Reaktionskolonne mit einem oder mehreren Zwischenkondensatoren ausgestattet ist, wird die durch Kondensation in dem oder den Zwischenkondensator(en) gewonnene Kondensationswärme erfindungsgemäß ebenfalls direkt oder indirekt, ganz oder teilweise wieder in das Verfahren zurückgeführt. Unter direkter Rückführung der Kondensationswärme in das Verfahren ist im Rahmen der Erfindung zu verstehen, dass diese Kondensationswärme ohne zwischengeschaltetes Heizmedium in das Verfahren zurückgeführt wird, z.B. zur Erwärmung entweder eines oder mehrerer Ströme oder zur Erwärmung eines oder mehrerer Kolonnenabschnitte innerhalb des Verfahrens. Dies kann beispielsweise in einem Wärmetauscher erfolgen. Vorzugsweise ist dabei entweder ein solcher Wärmeaustauscher mit dem Zwischenkondensator kombiniert. Unter indirekter Rückführung der Kondensationswärme in das Verfahren ist im Rahmen der Erfindung zu verstehen, dass mit der gewonnen Kondensationswärme zunächst ein Heizmedium erzeugt wird, welches zur Rückführung der Kondensationswärme in das Verfahren dient. Mit diesem Heizmedium können beispielsweise ein oder mehrere Ströme oder einer oder mehrere Kolonnenabschnitte innerhalb des Verfahrens erwärmt werden. Als Heizmedium kommen Gase, Dämpfe oder Flüssigkeiten in Frage, bevorzugt dampfförmige oder flüssige technische Wärmeträgermedien wie beispielsweise Wasser, Wärmeträger auf Mineralölbasis oder synthetische Wärmeträger (z.B. Diphyl^{™}, Marlotherm^{®}). Besonders bevorzugte Heizmedien sind Wasser oder Wasserdampf.

Erfindungsgemäß wird die durch Kondensation in dem oder den Kondensator(en) der weiteren Reaktionskolonne(n), vorzugsweise der zweiten Reaktionskolonne gewonnene Kondensationswärme, direkt oder indirekt, ganz oder teilweise für die Trennung des bei der Reaktion eingesetzten Dialkylcarbonats vom Reaktionsalkohol verwendet.

Besonders bevorzugt wird die durch Kondensation in dem oder den Kondensator(en) der weiteren Reaktionskolonne(n), vorzugsweise der zweiten Reaktionskolonne, und dem oder den gegebenenfalls vorhandenen Zwischenkondensator(en) der ersten Reaktionskolonne gewonnene Kondensationswärme direkt oder indirekt, ganz oder teilweise zur Trennung des Dialkylcarbonats vom während der Reaktion gebildeten Alkylalkohols verwendet.

Weiterhin bevorzugt wird die durch Kondensation in dem oder den Kondensator(en) der weiteren Reaktionskolonne(n), vorzugsweise der zweiten Reaktionskolonne, und/oder dem oder den gegebenenfalls vorhandenen Zwischenkondensator(en) der ersten Reaktionskolonne gewonnene Kondensationswärme direkt oder indirekt, teilweise zur Trennung des Dialkylcarbonats vom während der Reaktion gebildeten Alkylalkohols und teilweise zur Verdampfung des in die erste Reaktionskolonne eingeleiteten Dialkylcarbonats verwendet.

Besonders bevorzugt wird die durch Kondensation in dem oder den gegebenenfalls vorhandenen Zwischenkondensator(en) der ersten Reaktionskolonne direkt oder indirekt, ganz oder teilweise zur Verdampfung des in die erste Reaktionskolonne eingeleiteten Dialkylcarbonats verwendet.

In bevorzugten Ausführungsformen des erfindungsgemäßen Verfahrens wird die durch Kondensation in dem oder den Zwischenkondensator(en) der ersten Reaktionskolonne gewonnene Kondensationswärme direkt oder indirekt, ganz oder teilweise zur Verdampfung des in die erste Reaktionskolonne eingeleiteten Dialkylcarbonats verwendet und die durch Kondensation in dem oder den Kondensator(en) der weiteren Reaktionskolonne(n) gewonnene Kondensationswärme direkt oder indirekt, ganz oder teilweise zur Trennung des Dialkylcarbonats vom während der Reaktion gebildeten Alkylalkohols verwendet.

Im erfindungsgemäßen Verfahren fallen bei der Umesterung und/oder Disproprotionierung in der ersten Umesterunsgkolonne und/oder der oder den weiteren Reaktionskolonnen Ströme während der Reaktion gebildeter Alkylalkohol (Reaktionsalkohol) sowie nicht umgesetztes oder während der Reaktion gebildetes Dialkylcarbonat an und werden vorzugsweise in einem oder mehreren Strömen im Gemisch entnommen. Dieses in den Reaktionskolonnen nicht umgesetzte oder während der Reaktion gebildete Dialkylcarbonat wird erfindungsgemäß ganz oder teilweise in wenigstens einem weiteren Verfahrensschritt enthaltend wenigstens eine Destillationskolonne vom während der Reaktion gebildeten Alkylalkohol (Reaktionsalkohol) getrennt. Bevorzugt wird wenigstens ein Strom enthaltend nicht umgesetztes oder während der Reaktion gebildetes Dialkylcarbonat und während der Reaktion gebildeten Alkylalkohol am Kopf der ersten Reaktionskolonne entnommen und zur Auftrennung wenigstens einem weiteren Verfahrensschritt enthaltend wenigstens eine Destillationskolonne zugeführt.

Bevorzugt wird das am Kopf der ersten Reaktionskolonne entnommene Dampfgemisch enthaltend Dialkylcarbonat und während der Reaktion gebildetem Alkylalkohol nach Kondensation am Kopf der ersten Reaktionskolonne ganz oder teilweise wenigstens einem weiteren Verfahrensschritt enthaltend wenigstens eine Destillationskolonne zur Trennung von Dialkylcarbonat und Alkylalkohol zugeführt

Die Trennung Dialkylcarbonats und des Reaktionsalkohols erfolgt bevorzugt destillativ in einer oder mehreren Destillationskolonnen oder in einer Kombination aus Destillation und Membrantrennung - im Folgenden als Hybridprozess - bezeichnet.

Bilden Reaktionsalkohol und Dialkylcarbonat ein Azeptrop (z.B. Methanol und Dimethylcarbonat) so wird bevorzugt ein wenigstens zweistufiges Verfahren wie beispielsweise ein Zweidruckverfahren, eine Extraktivdestillation, eine Heteroazeotropdestillation mit einem niedrigsiedenden Schleppmittel oder ein Hybridverfahren verwendet. Besonders bevorzugt wird das Zweidruckverfahren oder ein Hybridverfahren angewendet. Ganz besonders bevorzugt wird das Zweidruckverfahren angewendet. Solche Verfahren sind dem Fachmann grundsätzlich bekannt (vgl. z.B. Ullmann's Encyclopedia of Industrial Chemistry, Vol. 7, 2007, Kap. 6.4. und 6.5.; Chemie Ingenieur Technik (67) 11 / 95).

Bilden Reaktionsalkohol und Dialkylcarbonat kein Azeotrop (z.B. Ethanol und Diethylcarbonat), so erfolgt die Trennung bevorzugt in einer einzelnen Destillationskolonne.

Bilden Reaktionsalkohol und Dialkylcarbonat ein Azeptrop, so weist das Destillat einer ersten Destillationskolonne des Verfahrensschritts zur Trennung von Dialkylcarbonat und Alkylalkohol (Reaktionsalkohol) vorzugsweise nahezu azeotrope Zusammensetzung auf. In diesem Fall wird dieses bevorzugt in einem Zweidruckverfahren wenigstens einer weiteren Destillationskolonne zugeführt die bei einem Betriebsdruck arbeitet, der unter dem der ersten Destillationskolonne liegt. Durch den unterschiedlichen Betriebsdruck verschiebt sich die Lage des Azeotrops hin zu geringeren Anteilen an Reaktionsalkohol. Man erhält als Sumpfprodukt dieser zweiten oder weiteren Destillationskolonne(n) Reaktionsalkohol in einer Reinheit von 90 bis 100 Gew.-%, bezogen auf das Gesamtgewicht des isolierten Sumpfproduktes, und als Destillat eine nahezu azeotropes Gemisch. Die bei geringerem Betriebsdruck arbeitende zweite oder weitere(n) Destillationskolonne(n) wird in ganz besonders bevorzugten Ausführungsformen vorzugsweise mit der Kondensationswärme des oder der Kopfkondensator(en) der ersten Destillationskolonne betrieben.

Beim Zweidruckverfahren macht man sich die Druckabhängigkeit der azeotropen Zusammensetzung eines Zweistoffgemisches zunutze. Bei einem Gemisch aus Reaktionsalkohol (Alkylalkohol) und Dialkylcarbonat, wie beispielsweise Methanol und Dimethylcarbonat, verschiebt sich die azeotrope Zusammensetzung mit zunehmendem Druck zu höheren Reaktionsalkoholgehalten. Führt man ein Gemisch dieser beiden Komponenten einer Kolonne (Dialkylcarbonatkolonne) zu, wobei der Reaktionsalkoholgehalt unterhalb der für den Betriebsdruck dieser Kolonne entsprechende azeotropen Zusammensetzung liegt, so erhält man als Destillat ein Gemisch mit nahezu azeotroper Zusammensetzung und als Sumpfprodukt nahezu reines Dialkylcarbonat. Das so erhaltende azeotrope Gemisch wird einer weiteren Destillationskolonne (Alkylalkoholkolonne) zugeführt. Diese arbeitet bei einem im Vergleich zur Dialkylcarbonatkolonne geringeren Betriebsdruck. Dadurch verschiebt sich die Lage des Azeotrops hin zu geringeren Reaktionsalkoholgehalten. Hierdurch ist es möglich, das in der Dialkylcarbonatkolonne erhaltene azeotrope Gemisch in ein Destillat mit nahezu azeotroper Zusammensetzung und nahezu reinen Reaktionsalkohol zu trennen. Das Destillat der Alkylalkoholkolonne wird wieder der Dialkylcarbonatkolonne an geeigneter Stelle zugeführt.

Der Betriebsdruck der Alkylalkoholkolonne wird vorzugsweise so gewählt, dass man diese mit der Abwärme der Dialkylcarbonatkolonne betreiben kann. Der Betriebsdruck liegt dabei zwischen 0,1 und 1 bar vorzugsweise zwischen 0,3 und 1 bar. Der Betriebsdruck der Dialkylcarbonatkolonne liegt im Bereich von 1 bis 50 bar, vorzugsweise zwischen 2 und 20 bar.

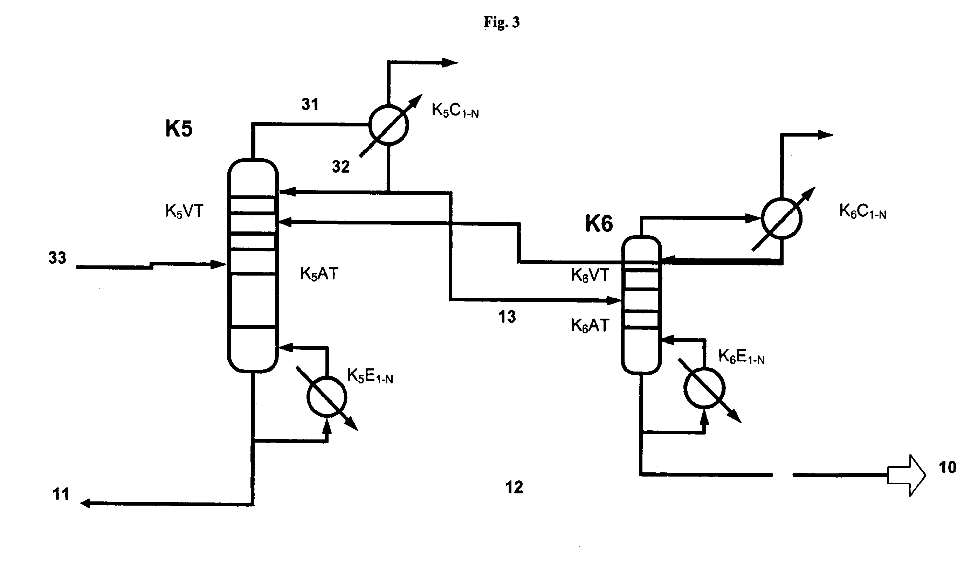

Eine beispielhafte Reaktionsführung bei der Trennung von Dialkylcarbonat und Reaktionsalkohol nach dem Zeidruckverfahren ist in Fig. 3 gezeigt.

Ein weiteres bevorzugtes Verfahren zur Trennung von Azeotropen aus Reaktionsalkohol und Dialkylcarbonat ist das Hybridverfahren. Beim Hybridverfahren erfolgt die Trennung eines Zweistoffgemisches mittels einer Kombination aus Destillation und Membranverfahren. Dabei macht man sich die Tatsache zunutze, dass man die Komponenten aufgrund ihrer polaren Eigenschaften und ihres unterschiedlichen Molekulargewichts mittels Membranen zumindest teilweise voneinander trennen kann. Im Falle eines Gemisches aus Reaktionsalkohol und Dialkylcarbonat, wie beispielsweise Methanol und Dimethylcarbonat, erhält man bei Verwendung geeigneter Membranen mittels Pervarporation oder Dampfpermeation ein Reaktionsalkoholreiches Gemisch als Permeat und ein an Reaktionsalkohol verarmtes Gemisch als Retentat. Führt man ein Gemisch dieser beiden Komponenten einer Kolonne (Dialkylcarbonatkolonne) zu, wobei der Reaktionsalkoholgehalt unterhalb der für den Betriebsdruck dieser Kolonne entsprechenden azeotropen Zusammensetzung liegt, so erhält man als Destillat ein Gemisch mit im Vergleich zum Zulauf deutlich erhöhtem Reaktionsalkoholgehalt und als Sumpfprodukt nahezu reines Dialkylcarbonat.

Im Falle eines Hybridverfahrens aus Destillation und Dampfpermeation wird das Destillat der Kolonne dampfförmig entnommen. Das so erhaltende dampfförmige Gemisch wird gegebenenfalls nach Überhitzung einer Dampfpermation zugeführt. Diese wird so betrieben, dass man auf der Retentatseite nahezu den Betriebsdruck der Kolonne und auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine Reaktionsalkoholreiche Fraktion mit einem Reaktionsalkoholgehalt von mindestens 70 Gew.-%, bevorzugt mindestens 90 Gew.-%, bezogen auf das Gesamtgewicht der Fraktion. Das Retentat, welches einen im Vergleich zum Destillat der Kolonne einen reduzierten Reaktionsalkoholanteil enthält, wird gegebenenfalls kondensiert und wieder der Destillationskolonne zugeführt.

Im Falle eines Hybridverfahrens aus Destillation und Pervaporation wird das Destillat der Kolonne flüssig entnommen. Das so erhaltende Gemisch wird gegebenenfalls nach Erhitzung einer Pervaporation zugeführt. Diese wird so betrieben, dass man auf der Retentatseite einen im Vergleich zur Kolonne identischen oder erhöhten Betriebsdruck auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine Reaktionsalkoholreiche dampfförmige Fraktion mit einem Reaktionsalkoholgehalt von mindestens 70 Gew.-%, bevorzugt mindestens 90 Gew.-%, bezogen auf das Gesamtgewicht der Fraktion. Das flüssige Retentat, welches einen im Vergleich zum Destillat der Kolonne reduzierten Reaktionsalkoholanteil erhält, wird wieder der Destillationskolonne zugeführt. Durch die Verdampfung des Permeats wird Wärme benötigt, die gegebenenfalls nicht in ausreichendem Maße im Zulaufstrom zur Pervaporation enthalten ist. Daher kann eine Membrantrennung mittels Pervarporation gegebenenfalls mit zusätzlichen Wärmetauschern beheizt werden, wobei diese integriert oder gegebenenfalls zwischen mehreren hintereinander geschalteten Pervaporationsschritten angebracht sind.

Die Trennung von Dialkylcarbonat und Reaktionsalkohol erfolgt im Falle eines Hybridverfahrens besonders bevorzugt mittels einer Kombination aus Destillation und Dampfpermeation.

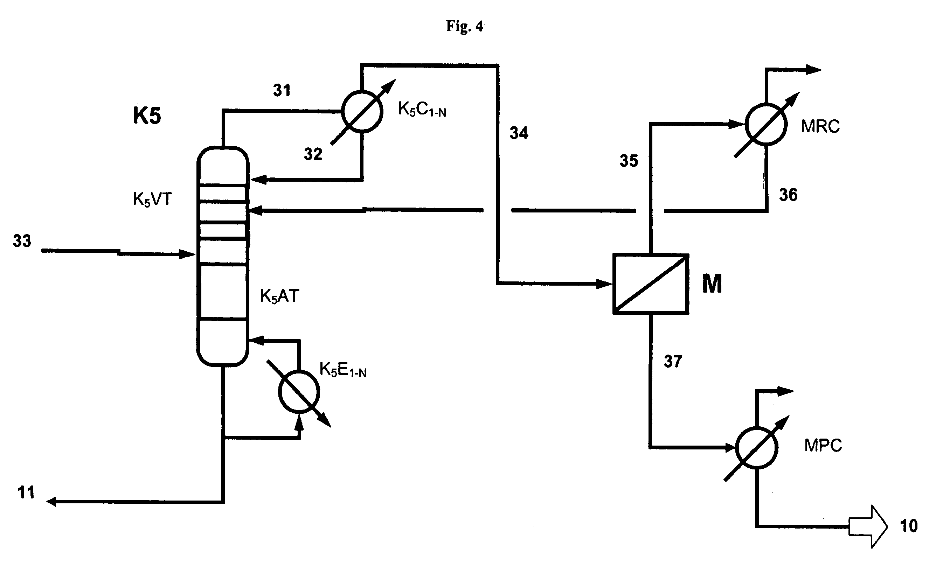

Eine beispielhafte Ausführung der Trennung von Dialkylcarbonat und Reaktionsalkohol nach dem Hybridverfahren mittels Dampfpermeation ist in Fig. 4 dargestellt.

Unabhängig vom gewählten Verfahren zur Trennung von Dialkylcarbonat und Reaktionsalkohol werden die Verfahrensbedingungen, wie Druck und Temperatur vorteilhafterweise so gewählt, das die die durch Kondensation in dem oder den Kondensator(en) der weiteren Reaktionskolonne(n) und/oder dem oder den gegebenenfalls vorhandenen Zwischenkondensator(en) der ersten Reaktionskolonne gewonnene Kondensationswärme effektiv genutzt werden kann.

Hierzu wird der Betriebsdruck und damit auch die Betriebstemperatur in der oder den Destillationskolonne(n) des Verfahrensschritts zur Trennung von Dialkylcarbonat und Alkylalkohol so eingestellt, dass die Destillationskolonne(n) ganz oder teilweise mit der Kondensationswärme in dem oder den Kondensator(en) der weiteren Reaktionskolonne(n) und/oder dem oder den gegebenenfalls vorhandenen Zwischenkondensator(en) der ersten Reaktionskolonne betrieben werden kann/können. Hierzu wird der Betriebsdruck in der oder den Destillationskolonne(n) des Verfahrensschritts zur Trennung von Dialkylcarbonat und Alkylalkohol so eingestellt, dass die Verdampungstemperatur im Sumpf der Destillationskolonne(n) des Verfahrensschritts zur Trennung von Dialkylcarbonat und Alkylalkohol unter der Kondensationstemperatur in dem oder den Kondensator(en) der weiteren Reaktionskolonne(n) und/oder dem oder den gegebenenfalls vorhandenen Zwischenkondensator(en) der ersten Reaktionskolonne liegt.

Die zur Trennung von Reaktionsalkohol und Dialkylcarbonat erforderliche Wärme wird bei einer Temperatur zwischen 100 und 300 °C, vorzugsweise zwischen 100 und 230, und besonders bevorzugt zwischen 120 und 200 °C zugeführt. Um eine Wärmeintegration mit dem Zwischenkondensator der ersten Reaktionskolonne oder den Kondensatoren der zweiten Reaktionskolonne effizient zu ermöglichen, wird die Kondensation in dem oder den Kondensator(en) der weiteren Reaktionskolonne(n) und/oder dem oder den gegebenenfalls vorhandenen Zwischenkondensator(en) der ersten Reaktionskolonne bei einer um 1 bis 100 °C, bevorzugt 2 bis 50 °C und besonders bevorzugt 5 bis 40 °C erhöhten Temperatur durchgeführt.

Die Kondensationswärme aus dem oder den Kondensator(en) der weiteren Reaktionskolonne(n) und/oder dem oder den gegebenenfalls vorhandenen Zwischenkondensator(en) der ersten Reaktionskolonne kann dabei beispielsweise ganz oder teilweise zum Vorheizen von Zuströmen zu der oder den Destillationskolonne(n) und/oder zur Erwärmung eines oder mehrerer Kolonnenabschnitte verwendet werden. In bevorzugten Ausführungsformen wird die Kondensationswärme aus dem oder den Kondensator(en) der weiteren Reaktionskolonne(n) und/oder dem oder den gegebenenfalls vorhandenen Zwischenkondensator(en) der ersten Reaktionskolonne teilweise zum Vorheizen des oder der Zuströme zu der oder den Destillationskolonne(n) des Verfahrensschritts zur Trennung von Dialkylcarbonat und Alkylalkohol und teilweise zum Verdampfen des Sumpfes in der oder den Destillationskolonne(n) verwendet. In einer ganz bevorzugten Ausführungsform des erfindungsgemäßen Verfahrens, in der am Kopf der zweiten Reaktionskolonne eine Kaskade von wenigstens zwei, bevorzugt drei Kopfkondensatoren verwendet wird, dient die Kondensationswärme aus dem ersten Kondensator dieser Kaskade zur Verdampfung des Sumpfproduktes der oder der ersten Destillationskolonne des Verfahrensschritts zur Trennung von Dialkylcarbonat und Alkylalkohol und die Kondensationswärme aus dem zweiten Kondensator der Kaskade zum Vorheizen des Zustroms zu der oder der ersten Destillationskolonne des Verfahrensschritts zur Trennung von Dialkylcarbonat und Alkylalkohol.

Die Destillationskolonne(n) verfügen bevorzugt über einen Verstärkungsteil mit 5 bis 40 theoretischen Stufen zur Aufkonzentrierung des Reaktionsalkohols und einen Abtriebsteil mit 5 bis 40 theoretischen Stufen zur Aufkonzentrierung des Dialkylcarbonates.

Das erfindungsgemäße Verfahren wird bevorzugt kontinuierlich durchgeführt.

Durch die Nutzung der Kondensationswärme aus dem oder den Kondensator(en) der weiteren Reaktionskolonne(n) und gegebenenfalls dem oder den Zwischenkondensatoren der ersten Reaktionskolonne kann die Trennung des Reaktionsalkohol von überschüssigem Dialkylcarbonat bei deutlich reduziertem Energieverbrauch durchgeführt werden. Die Kühlleistung in den Umesterungsschritten kann dabei in gleichem Maße reduziert werden. Ein wesentlicher Vorteil des erfindungsgemäßen Verfahrens gegenüber den Verfahren gemäß Stand der Technik liegt daher in der deutlichen Reduzierung des Energieverbrauchs bei der Herstellung von Diarylcarbonaten bzw. Alkylarylcarbonaten. Gleichzeitig kann das Verfahren mit einfachem apparativem Aufwand durchgeführt werden, da aufgrund der Verwendung von Kolonnenanordnungen keine komplizierte Reaktoranordnung mit mehreren getrennten und in Serie geschalteten Reaktionszonen erforderlich ist.

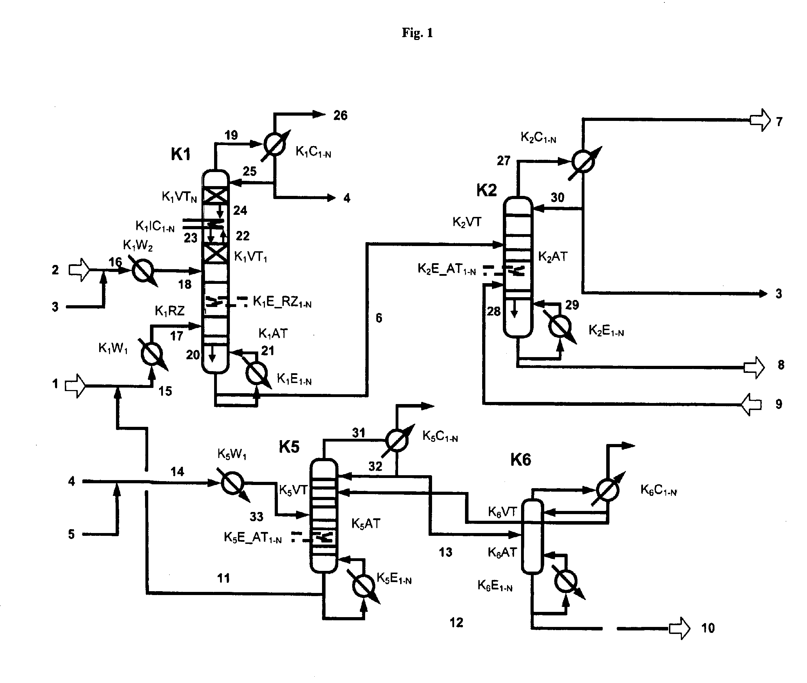

Das erfindungsgemäße Verfahren wird ausschnittsweise mit Hilfe der Fig. 1 beispielhaft erläutert. Die Fig. 1 zeigt das erfindungsgemäße Verfahren ohne etwaige nachfolgende Schritte wie zusätzliche Reaktionsschritte oder zusätzliche Reinigung in etwaigen weiteren Kolonnen.
Fig. 1 beschreibt einen ersten Umesterungsschritt mittels Reaktivrektifikation in einer ersten Reaktionskolonne mit Zwischenkondensator im Allgemeinen, einen zweiten Reaktionsschritt zur Umesterung bzw. zur Disproportionierung von Alkylarylcarbonat in einer zweiten Reaktionskolonne und eine Trennung des in der ersten Reaktionskolonne als Kopfprodukt anfallenden Gemisches enthaltend Dialkylcarbonat und Reaktionsalkohol in einem weiteren Verfahrensschritt enthaltend wenigstens eine Destillationskolonne.
Fig. 2 beschreibt eine besonders bevorzugte Ausführungsform einer ersten Reaktionskolonne (Reaktivrektifikationskolonne) mit externer Anordnung eines Zwischenkondensators und Kombination mit der Verdampfung des Dialkylcarbonats zur Rückführung der anfallenden Kondensationswärme.
Fig. 3 beschreibt eine bevorzugte Ausführungsform der Trennung von Dialkylcarbonat und Reaktionsalkohol nach dem Zweidruckverfahren.
Fig. 4 beschreibt eine bevorzugte Ausführungsform der Trennung von Dialkylcarbonat und Reaktionsalkohol nach dem Hybridverfahren.

Die Figuren dienen der beispielhaften Erläuterung der Erfindung und sind nicht als Beschränkung aufzufassen.

### In den Fig. 1 bis 4 bedeuten

- K1: Alkylarylcarbonat-Reaktionskolonne (AAC-Reaktionskolonne, erste Reaktionskolonne)
- K₁C_{1-N}: Kondensator(en) 1-N der AAC-Reaktionskolonne
- K₁E_{1-N}: Verdampfer 1 bis N der AAC-Reaktionskolonne
- K₁IC_{1-N}: Zwischenkondensator(en) 1 bis N der AAC-Reaktionskolonne
- K₁VT₁: unterer Verstärkungsteil der AAC-Reaktionskolonne
- K₁VT_{N}: oberer Verstärkungsteil der AAC-Reaktionskolonne
- K₁W₁: Vorwärmer/Verdampfer/Überhitzer für Dialkylcarbonat enthaltenden Strom
- K₁W₂: Vorwärmer/Verdampfer für Eduktstrom mit aromatischer Hydroxyverbindung
- K₁RZ: Reaktionszone der AAC-Reaktionskolonne
- K₁E_RZ_{1-N}: Zwischenverdampfer 1 bis N im Bereich der Reaktionszone der AAC- Reaktionskolonne
- K2: Diarylcarbonat-Reaktionskolonne (DAC-Reaktionskolonne/ Zweite Reaktionskolonne)
- K₂C_{1-N}: Kondensator(en) 1 bis N der DAC-Reaktionskolonne
- K₂E_{1-N}: Verdampfer 1 bis N der DAC-Reaktionskolonne
- K₂VT: Verstärkungsteil der DAC-Reaktionskolonne
- K₂AT: Abtriebsteil & Reaktionszone der DAC-Reaktionskolonne
- K₂E_AT_{1-N}: Zwischenverdampfer im Abtriebsteil der 2. Reaktionskolonne
- K5: Dialkylcarbonat-Destillationskolonne (DAK)
- K₅VT: Verstärkungsteil der DAK
- K₅AT: Abtriebsteil der DAK
- K₅W₁: Vorwärmer/Verdampfer für Reaktionsalkohol und Dialkylcarbonat enthaltenden Strom
- K₅C_{1-N}: Kondensator(en) 1 bis N der DAK
- K₅E_{1-N}: Verdampfer 1 bis N der DAK
- K₅E_AT_{1-N}: Zwischenverdampfer im Abtriebsteil der DAK
- K6: Reaktionsalkohol-Destillationskolonne (RAK)
- K₆C_{1-N}: Kondensator(en) 1 bis N der RAK
- K₆E_{1-N}: Verdampfer 1 bis N der RAK
- K₆VT: Verstärkungsteil der RAK
- K₆AT: Abtriebsteil der RAK
- M: Membrantrennung (Dampfpermeation oder Pervaporation)
- MRC: Kondensator für Retentat nach der Membrantrennung
- MPC: Kondensator für Permeat nach der Membrantrennung

### Weiterhin sind in den Fig. 1 bis 4 die folgenden Stoffströme benannt

- 1: Eduktzustrom enthaltend Dialkylcarbonat
- 2: Eduktzustrom entaltend aromatische Hydroxyverbindung
- 3: Destillat der zweiten Reaktionskolonne
- 4: Destillat der ersten Reaktionskolonne
- 5: Strom enthaltend Dialkylcarbonat und Reaktionsalkohol
- 6: Sumpfprodukt der ersten Reaktionskolonne
- 7: Zwischensiederpurge
- 8: Sumpfprodukt der zweiten Reaktionskolonne
- 9: Strom enthaltend Alkylarylcarbonat und aromatische Hydroxyverbindung
- 10: Ausschleusung Reaktionsalkohol
- 11: Dialkylcarbonat enthaltender Strom aus Dialkylcarbonat-Destillationskolonne (K5)
- 12: Destillat der Reaktionsalkohol-Destillationskolonne
- 13: Destillat der Dialkylcarbonat-Destillationskolonne
- 14: Strom enthaltend Dialkylcarbonat und Reaktionsalkohol
- 15: Dialkylcarbonat enthaltender Strom zur ersten Reaktionskolonne
- 16: Aromatische Hydroxyverbindung enthaltender Strom zur ersten Reaktionskolonne
- 17: Dialkylcarbonat enthaltender Strom nach Verdampfung
- 18: Strom mit aromatischer Hydroxyverbindung nach Erhitzung
- 19: Dampfstrom am Kopf der ersten Reaktionskolonne
- 20: Flüssiger Ablauf aus dem Abtriebsteil der ersten Reaktionskolonne
- 21: Dampf-Flüssigkeitsgemisch aus Sumpfverdampfer der ersten Reaktionskolonne
- 22: Dampfgemisch aus unterem Verstärkungsteil der ersten Reaktionskolonne
- 23: Kondensat des oder der Zwischenkondensatoren der ersten Reaktionskolonne
- 24: Ablauf Flüssigkeitsgemisch aus oberem Verstärkungsteil der ersten Reaktionskolonne
- 25: Rücklauf der ersten Reaktionskolonne
- 26: Restliches Dampfgemisch aus Kondensation der ersten Reaktionskolonne
- 27: Dampfstrom am Kopf der zweiten Reaktionskolonne
- 28: Ablauf Flüssigkeitsgemisch aus Reaktionszone oder gegebenenfalls Abtriebsteil der zweiten Reaktionskolonne
- 29: Dampf-Flüssigkeitsgemisch aus Sumpfverdampfer der zweiten Reaktionskolonne
- 30: Rücklauf der zweiten Reaktionskolonne
- 31: Dampfstrom am Kopf der Destillationskolonne (K5)
- 32: Rücklauf der Destillationskolonne (K5)
- 33: Zulaufgemisch zur Destillationskolonne (K5)
- 34: Destillat der Destillationskolonne zur Membrantrennung (M)
- 35: Retentat Membrantrennung (M) zum Kondensator (MRC)
- 36: Flüssiges Retentat zur Destillationskolonne (K5)
- 37: Permeat der Membrantrennung (M) zum Kondensator (MPC)

Fig. 1 zeigt unter anderem eine erste Reaktionskolonne K1, in die die beiden Eduktströme, d.h. ein die aromatische Hydroxyverbindung enthaltender Strom 16 und ein das Dialkylcarbonat enthaltender Strom 15 im Bereich einer Reaktionszone RZ im Sinne eine Gegenstromumesterung gegeneinander geführt und zu Alkylarylcarbonaten und geringen Anteilen Diarlycarbonaten umgesetzt werden.

Der das Dialkylcarbonat enthaltende Strom 15 kann - insbesondere bei kontinuierlich geführten Verfahren - neben dem Dialkylcarbonat auch Teile der aromatischen Hydroxyverbindung, die bei der Reaktion anfallende aliphatische Hydroxyverbindung R¹-OH und/oder R²-OH (Reaktionsalkohol), sehr geringe Mengen des bei der Umesterung anfallenden Alkylarylcarbonats und/oder Diarylcarbonats und bei der Reaktion entstehende unerwünschte Nebenkomponenten enthalten. Der das Dialkylcarbonat enthaltende Strom 15 kann beispielsweise 0 bis 5 Gew.-%, bevorzugt 0.05 bis 3 Gew: % und besonders bevorzugt 0.05 bis 2 Gew: % des Reaktionsalkohols, 0 bis 40 Gew.-%, bevorzugt 0 bis 10 Gew.-%, besonders bevorzugt 0 bis 5 Gew.-% der aromatischen Hydroxyverbindung, 0 bis 5 Gew.-% Alkylarylcarbonat, 0 bis 5 Gew.-% Diarylcarbonat und 0 bis 5 Gew.-% sonstige bei der Reaktion anfallende Nebenverbindungen (wie z.B. Alkylarylether) oder bereits in den Edukten enthaltene Verunreinigungen, jeweils bezogen auf das Gesamtgewicht des Dialkylcarbonat enthaltenden Stromes, aufweisen. Vorzugsweise enthält der das Dialkylcarbonat enthaltende Strom 15 50 bis 100 Gew.-% Dialkylcarbonat, bezogen auf das Gesamtgewicht des Dialkylcarbonat enthaltenden Stromes, wobei sich die Anteile der einzelnen vorangehend genannten Komponenten zu 100 Gew.-% addieren. Der die aromatische Hydroxyverbindung enthaltende Strom 16 kann - insbesondere bei kontinuierlich geführten Verfahren - neben der aromatischen Hydroxyverbindung auch Teile des Dialkylcarbonats, das bei der Umesterung anfallende Alkylarylcarbonat und/oder Diarylcarbonat, in sehr geringen Mengen den Reaktionsalkohol und bei der Reaktion entstehende unerwünschte Nebenprodukte enthalten. Beispielsweise kann der Gehalt des Dialkylcarbonats 0 bis 50 Gew.-%, der Gehalt des Reaktionsalkohols 0 bis 10 Gew.-%, bevorzugt 0 bis 5 Gew.-%, der Gehalt des Alkylarylcarbonats und des Diarylcarbonats jeweils 0 bis 10 Gew.-%, bevorzugt 0 bis 5 Gew.-% und der Gehalt der unerwünschten Nebenprodukte 0 bis 5 Gew.-%, bevorzugt 0 bis 1 Gew.-%, jeweils bezogen auf das Gesamtgewicht des die aromatische Hydroxyverbindung enthaltenden Stromes, betragen. Mit dem die aromatische Hydroxyverbindung enthaltenden Strom 16 kann zudem der Katalysator in die Reaktionskolonne eingespeist werden. In diesem Fall beträgt der Gehalt an Katalysator bevorzugt 0 bis 5 Gew.-%, bezogen auf das Gesamtgewicht des die aromatische Hydroxyverbindung enthaltenden Stromes. Vorzugsweise enthält der die aromatische Hydroxyverbindung enthaltende Strom 16 50 bis 100 Gew.-% aromatische Hydroxyverbindung, bezogen auf das Gesamtgewicht des die aromatische Hydroxyverbindung enthaltenden Stromes, wobei sich die Anteile der einzelnen vorangehend genannten Komponenten zu 100 Gew.-% addieren.

Der das Dialkylcarbonat enthaltende Strom 15 wird vor Einleiten in die Kolonne K1 teilweise oder vollständig verdampft und gegebenenfalls überhitzt. Der die aromatische Hydroxyverbindung enthaltende Strom 16 wird vor Einleiten in die Kolonne K1 erhitzt und dabei gegebenenfalls teilweise verdampft. Die Eduktströme 17 und 18, jeweils nach Verdampfung und gegebenenfalls Überhitzung bzw. nach Erhitzen, werden in der Reaktionszone RZ im Gegenstrom zueinander geführt, d.h. der die aromatische Hydroxyverbindung enthaltende Strom 18 wird am oberen Ende der Reaktionszone RZ in erhitzter, vorwiegend flüssiger Form und der das Dialkylcarbonat enthaltende Strom 17 wird am unteren Ende der Reaktionszone vorwiegend gasförmig oder gegebenenfalls leicht überhitzt zugeführt. Die bei der Reaktion anfallende aliphatische Hydroxyverbindung R¹-OH und/oder R²-OH wird zusammen mit noch nicht umgesetzten Dialkylcarbonat am Kopf der Kolonne dampfförmig abgezogen (19) und das schwererflüchtige Alkylarylcarbonat zusammen mit noch nicht umgesetzten Mengen der aromatischen Hydroxyverbindung, Diarylcarbonat und gegebenenfalls weiteren schwerflüchtigen Verbindungen als flüssiger Strom am Fuß der Kolonne K1 entnommen (6). Die zur Einstellung des gewünschten Temperaturprofils erforderliche Energie kann unter Anderem am Sumpf der Kolonne durch einen oder mehrere Verdampfer K₁E_{1-N} erfolgen. Hierzu wird das aus dem Abtriebsteil K₁AT, bzw. bei nicht vorhandenem Abtriebsteil aus der Reaktionszone K₁RZ, ablaufende flüssige Gemisch (20) teilweise verdampft. Je nach Verdampferausführung erhält man am Austritt des Verdampfers nur Dampf oder ein Dampf-Flüssigkeitsgemisch (Strom 21). Der im Strom 21 enthaltene Dampf wird dem Abtriebsteil (K₁AT) von unten, oder falls kein Abtriebsteil vorhanden ist, der Reaktionszone K₁RZ von unten zugeführt. Im Bereich der Reaktionszone kann durch zusätzliche Zwischenverdampfer K₁E_RZ_{1-N} Wärme zugeführt werden. Im zwischen Reaktionszone K₁RZ und Verdampfer K₁E_{1-N} angebrachten Abtriebsteil K₁AT erfolgt eine Aufkonzentrierung des gebildeten Alkylarylcarbonats und des Diarylcarbonats, wobei bereits in diesem Teil der Kolonne K1 durch die Verarmung an Dialkylcarbonat die Disproportionierungsreaktion von Alkylarylcarbonat zu Diarylcarbonat in verstärktem Maße einsetzt.

In einem oder mehreren zwischen dem/den Kondensator/en K₁C_{1-N} und Reaktionszone K₁RZ befindlichen Verstärkungsteil(en) erfolgt die Aufkonzentrierung der bei der Reaktion gebildeten aliphatischen Hydroxyverbindung (Reaktionsalkohol) und des überschüssigen Dialkylcarbonats. Dabei soll ein Gehalt an aromatischer Hydroxyverbindung(en) im Destillat 4 von 0 bis 40 Gew.-% bevorzugt 0 bis 10 Gew.-%, besonders bevorzugt von 0 bis 5 Gew.-%, bezogen auf Gesamtgewicht des Destillats 4, eingestellt werden. Der Verstärkungsteil ist in mindestens zwei Sektionen, den oberen und den unteren Verstärkungsteil, unterteilt, wobei sich zwischen dem oberen Verstärkungsteil K₁VT_{N} und dem unteren Verstärkungsteil K₁VT₁ ein oder mehrere Zwischenkondensator(en) K₁IC_{1-N}, bevorzugt wenigstens ein Zwischenkondensator K₁IC₁, befindet. Diese(r) Zwischenkondensator(en) K₁IC_{1-N} bzw. dieser Zwischenkondensator K₁IC₁ kondensiert einen Teil der aus dem unteren Verstärkungsteil K₁VT₁ aufsteigenden Dämpfe 22. Das in den oder die Zwischenkondensator(en) K₁IC_{1-N}, bevorzugt wenigstens einen Zwischenkondensator K₁IC₁, eintretende dampfförmige Gemisch 22 enthält vorzugsweise 10 bis 80 Gew.-% an aromatischer Hydroxyverbindung. Die Kondensationstemperatur in dem oder den Zwischenkondensator(en) K₁IC₁-_{N} ist daher aufgrund verhältnismäßig hoher Mengen an aromatischer Hydroxyverbindung deutlich höher im Vergleich zur Kondensationstemperatur im Kopfkondensator K₁C_{1-N} (N: Kondensator ist gegebenenfalls mehrstufig). Je nach Betriebsdruck und Lage des Konzentrationsprofils kann die Kondensationstemperatur in dem oder den Zwischenkondensator(en) bevorzugt im Bereich von 100 bis 300°C, besonders bevorzugt von 120 bis 250°C, ganz besonders bevorzugt von 150 bis 240°C und im Kopfkondensator bevorzugt im Bereich von 0 bis 250°C, besonders bevorzugt von 40 bis 200°C liegen. Das in dem oder den Zwischenkondensator(en) K₁IC_{1-N} anfallende Kondensat 23 sowie die aus dem darüber liegenden oberen Verstärkungsteil K₁VT_{N} ablaufende Flüssigkeit 24 wird auf den unteren Verstärkungsteil K₁VT₁ geleitet. Das dampfförmige Gemisch nach dem oder den Zwischenkondensator(en) gelangt in den oberen Verstärkungsteil K₁VT_{N}. Der aus dem oberen Verstärkungsteil K₁VT_{N} kommende Dampf 19 wird in dem oder den Kondensator(en) K₁C_{1-N} weitestgehend kondensiert, wobei das Kondensat teilweise wieder als Rücklauf dem oberen Verstärkungsteil K₁VT_{N} zugeführt wird (25) und teilweise als Destillatstrom 4 entnommen wird. Der Destillatstrom 4 enthält im Wesentlichen das im Überschuss eingesetzte Dialkylcarbonat und den entsprechenden bei der Reaktion entstehenden Alkylalkohol R¹-OH und/oder R²-OH (Reaktionsalkohol) und gegebenenfalls geringe Mengen der aromatischen Hydroxyverbindung. Das restliche Dampfgemisch aus dem oder den Kondensator(en) K₁C_{1-N} wird als Dampfstrom 26 entnommen.

Die in dem oder den Zwischenkondensator(en) K₁IC_{1-N}, bevorzugt wenigstens dem Zwischenkondensator K₁IC₁, frei werdende Kondensationswärme kann wie vorangehend für das erfindungsgemäße Verfahren beschrieben direkt oder indirekt wieder in das Verfahren zurückgeführt werden (In Fig. 1 nicht gezeigt).

In einer bevorzugten Ausführungsform des erfindungsgemäßen Verfahrens wird die in dem oder den Zwischenkondensator(en) K₁IC_{1-N}, bevorzugt wenigstens dem Zwischenkondensator K₁IC₁, anfallende Kondensationswärme zur Erhitzung eines Wärmeträgermediums verwendet. Dieses wiederum wird zur Verdampfung und Überhitzung des bei der Gegenstromumesterung in der Reaktionskolonne K1 eingesetzten Dialkylcarbonat enhaltenden Stromes 15 verwendet. Bei dieser bevorzugten Ausführungsform handelt es sich um eine indirekte Nutzung der Kondensationswärme.

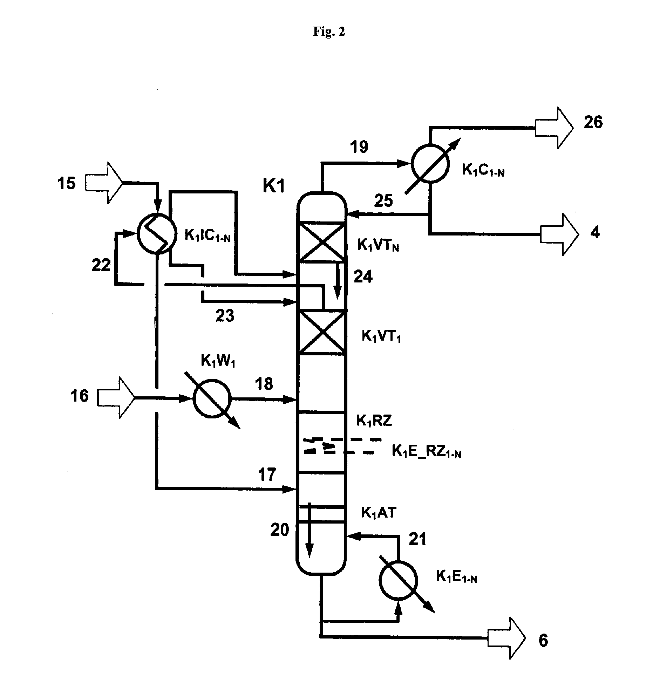

Eine andere bevorzugte Ausführungsform der Umesterung in der ersten Reaktionskolonne in Gegenwart wenigstens eines Zwischenkondensators wird in Fig. 2 gezeigt. Hierbei werden der oder die Zwischenkondensator(en) außerhalb der ersten Reaktionskolonne ausgeführt. Die Erhitzung, Verdampfung und gegebenenfalls Überhitzung des Dialkylcarbonat enhaltenden Stromes 15 erfolgt ebenfalls im Zwischenkondensator. Dabei wird das dampfförmige Gemisch 22 des unteren Verstärkungsteils K₁VT₁ zu dem oder den Zwischenkondensator(en) K₁IC_{1-N}, bevorzugt zu wenigstens einem Zwischenkondensator K₁IC₁, geleitet, wo dieses teilweise kondensiert. Das dabei anfallende Kondensat 23 wird wieder dem unteren Verstärkungsteil K₁VT₁ zugeführt und die nicht kondensierten Dämpfe in den oberen Verstärkungsteil K₁VT_{N} geleitet. Ansonsten entspricht das in Fig. 2 gezeigte Verfahren dem in Fig. 1 dargestellten. Die vorangehenden Erläuterungen für Fig. 1 gelten daher analog.

Gemäß Fig. 1 wird das Sumpfprodukt 6 der ersten Reaktionskolonne K1 einer zweiten Reaktionskolonne K2 zugeführt. Dieses kann 0 bis 60 Gew.-% Diarylcarbonat, 5 bis 80 Gew.-% Alkylarylcarbonat, 5 bis 95 Gew.-% der aromatischen Hydroxyverbindung, 1 bis 80 Gew.-% Dialkylcarbonat, 0 bis 5 Gew.-% Katalysator und 0 bis 5 Gew.-% sonstige bei der Reaktion anfallende Nebenverbindungen (wie z.B. Alkylarylether) oder bereits in den Edukten enthaltene Verunreinigungen, jeweils bezogen auf das Gesamtgewicht des Sumpfproduktstromes 6, aufweisen. Die Prozentanbgaben beziehen sich auf das Gesamtgewicht des Sumpfproduktstromes 6, wobei sich die Anteile der einzelnen vorangehend genannten Komponenten zu 100 Gew.-% addieren.

Zusätzlich zum Sumpfprodukt der ersten Reaktionskolonne können darüber hinaus mindestens noch ein weiterer Alkylarylcarbonat enthaltender Strom 9 der zweiten Reaktionskolonne zugeführt werden. Dieser Strom 9 kann beispielsweise aus einem weiteren Aufarbeitungsschritt zur Aufreinigung des Diarylcarbonat, wie z.B. einer Destillationskolonne, stammen.

Dieser kann 0 bis 10 Gew.-% Diarylcarbonat, 10 bis 100 Gew.-% Alkylarylcarbonat, 0 bis 90 Gew.-% der aromatischen Hydroxyverbindung, 0 bis 20 Gew.-% Dialkylcarbonat und 0 bis 20 Gew.-% sonstige bei der Reaktion anfallende Nebenverbindungen (wie z.B. Alkylarylether) oder bereits in den Edukten enthaltene Verunreinigungen, jeweils bezogen auf das Gesamtgewicht des Dialkylcarbonat enthaltenden Stromes, aufweisen. Die Prozentanbgaben beziehen sich auf das Gesamtgewicht des Stromes 9, wobei sich die Anteile der einzelnen vorangehend genannten Komponenten zu 100 Gew.-% addieren.

Die Ströme 6 und 9 werden der Reaktionszone K₂AT der zweiten Reaktionskolonne zugeführt.

Die bei der Umesterung anfallende Reaktionsalkohol R¹-OH und/oder R²-OH wird zusammen mit noch nicht umgesetzten oder bei der Disproportionierung freigesetztem Dialkylcarbonat und nicht umgesetzter aromatischer Hydroxyverbindung am Kopf der Kolonne K2 dampfförmig abgezogen (27) und das schwererflüchtige Diarylcarbonat zusammen mit noch nicht umgesetzten Mengen der aromatischen Hydroxyverbindung, Alkylarylcarbonat und gegebenenfalls weiteren schwerflüchtigen Verbindungen als flüssiger Strom am Fuß der zweiten Reaktionskolonne K2 entnommen (8).

Die zur Einstellung des gewünschten Temperaturprofils erforderliche Energie kann unter anderem am Sumpf der Kolonne durch einen oder mehrere Verdampfer K₂E_{1-N} erfolgen. Hierzu wird das aus der Reaktionszone, ablaufende flüssige Gemisch (28) teilweise verdampft. Je nach Verdampferausführung erhält man am Austritt des Verdampfers nur Dampf oder ein Dampf-Flüssigkeitsgemisch (Strom 29). Der im Strom 29 enthaltene Dampf wird dem Abtriebsteil (K₂AT), welcher gleichzeitig auch als Reaktionszone fungiert und aus mehreren Sektionen besteht von unten zugeführt. Im Bereich der Reaktionszone kann durch zusätzliche Zwischenverdampfer K₂E_AT_{1-N} Wärme zugeführt werden. In der Reaktionszone K₂AT und im Verdampfer K₂E_{1-N} erfolgt sowohl Reaktion (Umesterung und/oder bevorzugt Disproportionierung) als auch Abtrennung der gebildeten leichtsiedenden Reaktionsprodukte (Reaktionsalkohol und Dialkylcarbonat) und der aromatischen Hydroxyverbindung.

In einem zwischen dem/den Kondensator/en K₂C_{1-N} und Reaktionszone K₂AT befindlichen Verstärkungsteil K₂VT wird der Gehalt an schwersiedenden Verbindungen wie beispielsweise Alkylarylcarbonat oder Diarylcarbonat reduziert. Dabei wird vorzugsweise ein Gehalt an Alkylarylcarbonat im Destillat 3 von 0 bis 20 Gew.-%, bevorzugt 0 bis 5 Gew.-%, besonders bevorzugt von 0 bis 2 Gew.-%, bezogen auf Gesamtgewicht des Destillats 3, eingestellt. Der Verstärkungsteil kann analog zur ersten Reaktionskolonne mit einem oder mehreren Zwischenkondensatoren ausgeführt werden. In der in Fig. 2 dargestellten bevorzugten Ausführungsform wird der Verstärkungsteil der K2 jedoch ohne Zwischenkondensator(en) ausgeführt.

Der oder die Kondensator(en) K₂C_{1-N}, in einer ganz besonders bevorzugten Ausführungsform eine Kaskade von Kondensatoren, am Kopf der K2 kondensieren einen Teil der aus dem Verstärkungsteil K₂VT aufsteigenden Dämpfe 27. Das in den oder die Kondensator(en) K₂C_{1-N} eintretende dampfförmige Gemisch 27 enthält vorzugsweise 10 bis 90 Gew.-% an aromatischer Hydroxyverbindung. Die Kondensationstemperatur in dem oder den Kondensator(en) K₂C_{1-N} ist daher aufgrund verhältnismäßig hoher Mengen an aromatischer Hydroxyverbindung hoch. Je nach Betriebsdruck und Zusammensetzung des dampfförmigen Gemisches 27 kann die Kondensationstemperatur in dem oder den Kondensator(en) bevorzugt im Bereich von 100 bis 300°C, besonders bevorzugt von 120 bis 250°C, ganz besonders bevorzugt von 150 bis 240°C liegen. Das Kondensat wird teilweise wieder als Rücklauf 30 dem Verstärkungsteil K₂VT zugeführt und teilweise als Destillatstrom 3 entnommen.

Der Destillatstrom 3 enthält im Wesentlichen aromatische Hydroxyverbindungen und geringe Mengen an Reaktionsalkohol, bevorzugt 0 bis 5 Gew.-%.

Das Destillat der ersten Reaktionskolonne (4) wird gegebenenfalls zusammen mit weiteren, Reaktionsalkohol und Dialkylcarbonat enhaltenden Strömen (5 und/oder 12) gegebenenfalls nach Erhitzung und/oder teilweiser Verdampfung einer Destillationskolonne K5 (Dialkylcarbonat-Destillationskolonne) zur Trennung des Dialkylcarbonats vom gebildeten Reaktionsalkohols zugeführt, wobei der erhaltene Dialkylcarbonat enthaltende Strom 11 wieder dem Dialkylcarbonat enthaltenden Zustrom 15 der ersten Reaktionskolonne zugeführt und der abgetrennte Reaktionsalkohol aus dem Verfahren ausgeschleust wird (10). Strom 5 kann dabei beispielsweise aus weiteren Renigungs- bzw. Nebenproduktabtrennungsschritten stammen.

Bilden Reaktionsalkohol und Dialkylcarbonat ein Azeotrop, so erhält man als Destillat (13) der Destillationskolonne K5 vorzugsweise ein annähernd azeotropes Gemisch. Für eine vollständige Trennung von Reaktionsalkohol und Dialkylcarbonat ist daher mindestens ein weiterer Trennschritt erforderlich.

Bilden Reaktionsalkohol und Dialkylcarbonat kein Azeotrop, so erhält man als Destillat vorzugsweise Reaktionsalkohol mit einem Gehalt von 95 bis 100 Gew.-%.

Als Sumpfprodukt der Destillationskolonne K5 wird ein Gemisch enthaltend Dialkylcarbonat mit weniger als 5 Gew.-% Reaktionsalkohol entnommen.

Die Dialkylcarbonat-Destillationskolonne K5 verfügt über einen Verstärkungsteil mit bevorzugt 5 bis 40 theoretischen Stufen zur Aufkonzentrierung des Reaktionsalkohols und einen Abtriebsteil mit bevorzugt 5 bis 40 theoretischen Stufen zur Aufkonzentrierung des Dialkylcarbonates.

Die für die Destillation in der Dialkylcarbonat-Destillationskolonne erforderliche Energie kann unter anderem am Sumpf der Kolonne durch einen oder mehrere Verdampfer K₅E_{1-N} erfolgen. Im Bereich des Abtriebsteils K₅AT kann durch zusätzliche Zwischenverdampfer K₅E_AT_{1-N} Wärme zugeführt werden.

Der oder die Kondensator(en) K₅C_{1-N} kondensieren die aus dem Verstärkungsteil K₅VT aufsteigenden Dämpfe 31. Das Kondensat wird teilweise wieder als Rücklauf 32 dem Verstärkungsteil K₅VT zugeführt und teilweise als Destillatstrom 13 entnommen.

Der Destillatstrom 13 enthält Reaktionsalkohol und Dialkylcarbonat in nahezu azeotroper Zusammensetzung. Bilden Reaktionsalkohol und Dialkylcarbonat kein Azeotrop, so erhält man als Destillat nahezu reinen Reaktionsalkohol.

Der Betriebsdruck in der Dialkylcarbonat-Destillationskolonne (K5) wird so eingestellt, dass die Kolonne mit Abwärme aus dem Umesterungsprozess betrieben werden kann. Hierzu wird die Kondensationswärme aus dem Zwischenkondensator der ersten Reaktionskolonne und/oder dem oder den Kondensator(en) der zweiten Reaktionskolonne genutzt. Der Betriebsdruck in der Kolonne K5 wird so eingestellt, dass die Verdampungstemperatur im Sumpf der Kolonne K5 unter der Kondensationstemperatur im Zwischenkondensator der ersten Reaktionskolonne und/oder dem oder den Kondensatoren der zweiten Reaktionskolonne liegt.

Bilden Reaktionsalkohol und Dialkylcarbonat unter den Bedingungen in der Destillationskolonne K5 ein Azeotrop, so kann man dieses mittels Schleppmittel- oder Extrekativrektifikation, nach dem Zweidruckverfahren oder mittels einer Kombination aus Rektifikation und Membrantrennung getrennt werden. Besonders bevorzugt wird das Zweidruckverfahren zur Trennung des Reaktionsalkohol und des Dialkylcarbonat eingesetzt, welches anhand von Fig. 1 und 3 ebenfalls beispielhaft erläutert wird.

Hat das Destillat der Destillationskolonne K5 eine azeotrope Zusammensetzung, so wird dieses einer weiteren Kolonne (Reaktionsalkohol-Destillationskolonne (RAK); K6 in Fig. 1 und 3) zugeführt die bei einem Betriebsdruck arbeitet, der unter dem der Destillationskolonne K5 liegt. Durch den unterschiedlichen Betriebsdruck verschiebt sich die Lage des Azeotrops hin zu geringeren Anteilen an Reaktionsalkohol. Man erhält als Sumpfprodukt 10 der Destillationskolonne K6 Reaktionsalkohol mit einer Reinheit von 90 bis 100 Gew.-% und als Destillat der Kolonne K6 eine nahezu azeotropes Gemisch. Die bei geringerem Betriebsdruck arbeitende Kolonne K6 wird in einer besonders bevorzugten Ausführungsform mit der Kondensationswärme des oder der Kopfkondensator(en) der Kolonne K5 betrieben.

Die Reaktionsalkohol-Destillationskolonne (RAK) K6 verfügt über einen Verstärkungsteil K₆VT mit 5 bis 40 theoretischen Stufen zur Aufkonzentrierung des Reaktionsalkohols und einen Abtriebsteil K₆AT mit 5 bis 40 theoretischen Stufen zur Aufkonzentrierung des Dialkylcarbonates.

Weiterhin bevorzugt kann das Azeotrop aus Reaktionsalkohol und Dialkylcarbonat auch mittels eines Hybridverfahrens in Form einer Kombination aus Rektifikation und Membrantrennung getrennt werden (vgl. Fig. 4). Dabei wird das Destillat der K5 einer Membrantrennung M zugeführt, welche in ihren unterschiedlichen Ausführungsformen bereits vorangehend beschrieben wurde. Dabei wird auf der Permeatseite eine Reaktionsalkoholreiche Fraktion 37 mit einem Reaktionsalkoholgehalt von mindestens 70 Gew.-%, bevorzugt mindestens 90 Gew.-%, bezogen auf das Gesamtgewicht der Fraktion erhalten und im Kondensator MPC kondensiert. Das Retentat 35, welches einen im Vergleich zum Destillat der Kolonne K5 reduzierten Reaktionsalkoholanteil enthält, wird im Kondensator MRC kondensiert und vorzugsweise wieder der Destillationskolonne K5 zugeführt (36).

Die folgenden Beispiele dienen der beispielhaften Erläuterung der Erfmdung und sind nicht als Beschränkung aufzufassen.

### Beispiele

### Beispiel 1 (erfindungsgemäß)

In eine erste Reaktionskolonne enthaltend
- einem oberen Verstärkungsteil (K₁VT₂) mit 4 theoretischen Stufen
- einem Zwischenkondensator (K₁IC₁)
- einem unteren Verstärkungsteil (K₁VT₁) mit 4 theoretischen Stufen
- einer Reaktionszone (K₁RZ) mit 30 Reaktionsböden (Hold-up: 12 1), wobei 3 Böden mit Heizelementen (K₁E_RZ₁₋₃) ausgestattet sind und
- einem Abtriebsteil K₁AT mit 6 Böden (Hold-up: 121)
werden 400 kg/h eines Gemisches aus 85.4 Gew.-% Phenol, 9.2 Gew.-% Dimethylcarbonat, 3.2 Gew.-% Diphenylcarbonat, 1.5 Gew.-% Titantetraphenolat, 0.3 Gew.-% Anisol, 0.3 Gew.-% Methylphenylcarbonat und 0.1 Gew.-% Methanol am oberen Ende der Reaktionszone zudosiert. Am unteren Ende der Reaktionszone werden 539,6 kg/h eines um 5 °C überhitzten Dampfgemisches aus 98.8 Gew.-% Dimethylcarbonat, 0.9 Gew.-% Phenol, 0.2 Gew.-% Anisol und 0.1 Gew.-% Methanol zugeführt.

Dabei erhält man am Sumpf der Kolonne 456.9 kg/h ein Produktgemisch aus 51 Gew.-% Phenol, 27.3 Gew.-% MPC (124.7 kg/h), 11.9 Gew.-% DPC (54.3 kg/h), 8.1 Gew.-% DMC, 0.4 Gew.-% Anisol und 1.3 Gew.-% Titantetraphenolat.

Die erste Reaktionskolonne wird bei einem Kopfdruck (oberhalb von K₁VT₂) von 3.6 bar und einem Rücklaufverhältnis von 1.15 betrieben. Dabei wird im Sumpf der Kolonne eine Temperatur von 230 °C und in der Reaktionszone eine mittlere Reaktionstemperatur von 215 °C eingestellt. Ein Sumpfverdampfer K₁E₁ und Zwischenverdampfer K₁E_RZ₁ - K₁E_RZ₃ in der Reaktionszone werden mit Heizdampf bei einem Dampfdruck von 35 bar betrieben, wobei als Sumpfverdampfer K₁E₁ ein Naturumlaufverdampfer verwendet wird und als Zwischenverdampfer auf den Reaktionsböden integrierte Heizregister verwendet werden. Die Eintrittstemperatur in den Zwischenkondensator beträgt 205°C, die Austrittstemperatur 193 °C und die Kühlleistung 57 kW. Die bei der Zwischenkondensation anfallende Kondensationswärme kann zur Erzeugung von Heizdampf mit einem Heizdampfdruck von 8 bar (Tautemperatur: 170.4 °C) verwendet werden. Die erforderliche Heizleistung zur Verdampfung des Dimethylcarbonat enthaltenden Stromes beträgt 52 kW. Die Verdampfung und Überhitzung des Dimethylcarbonates erfolgt bei einer Temperatur von 135 bis 152 °C, wozu der im Zwischenkondensator verwendete Dampf problemlos verwendet werden kann.

Das Sumpfprodukt der ersten Reaktionskolonne wird einer zweiten Reaktionskolonne enthaltend
- einem Verstärkungsteil (K₂VT) mit 10 theoretischen Stufen
- einen Abtriebsteil inklusive Reaktionszone (K₂AT) mit 22 theoretischen Stufen
am oberen Ende der Reaktionszone (K₂AT) zugeführt.

Zusätzlich werden 81,9 kg/h eines Gemisches aus 69,9 Gew.-% Methylphenylcarbonat, 28.3 Gew.-% Phenol, 1.2 Gew.-% Dimethylcarbonat, 0,5 Gew.-% Diphenylether und 0,1 Gew.-% Diphenylcarbonat, in der unteren Hälfte des Abtriebsteils (K₂AT) zudosiert.

Dabei werden am Sumpf der zweiten Reaktionskolonne 236,6 kg/h eines Produktgemisches aus 62,8 Gew.-% Diphenylcarbonat, 24.2 Gew.-% Methylphenylcarbonat, 9.8 Gew.-% Phenol, 0.4 Gew.-% DMC, 2.6 Gew.-% Titantetraphenolat und 0.2 Gew.-% Diphenylether erhalten.

Ferner werden 238.2 kg/h flüssiges Destillat mit 83.5 Gew.-% Phenol, 15.5 Gew.-% Dimethylcarbonat, 0.6 Gew.-% Methylphenylcarbonat, 0.3 Gew.-% Anisol und 0.1 Gew.-% Methanol entnommen.

Das aus der zweiten Reaktionskolonne kommende Dampfgemisch wird nur teilweise kondensiert, so dass auch 59.5 kg/h eines dampfförmigen Produktstromes nach der Kondensation, zwecks Ausschleusung von mittelsiedenden Nebenverbindungen, insbesondere Anisol, entnommen werden. Dieser dampfförmige Produktstrom enthält 59.8 Gew.-% Dimethylcarbonat, 38.2 Gew.-% Phenol, 1.6 Gew.-% Methanol, 0.3 Gew.-% Anisol und 0,1 Gew.-% Methylphenylcarbonat.

Die zweite Reaktionskolonne wird bei einem Kopfdruck (oberhalb von K₂VT) von 1 bar und einem Rücklaufverhältnis von 0.65 betrieben. Durch die Verwendung strukturierter Packungen in Verstärkungs- und Abtriebsteil liegt der Druckverlust in der Kolonne unter 50 mbar. Das aus der Reaktionszone ablaufende Gemisch hat eine Temperatur von 198 °C und wird in einer zweistufigen Verdampfung zugeführt. Die Austrittstemperatur nach der ersten Verdampfungsstufe beträgt 209 und nach der zweiten Verdampferstufe 230 °C. Als Verdampfer werden in der ersten Stufe ein Naturumlaufverdampfer und in der zweiten Stufe ein Kettle-type-Verdampfer verwendet. Die gesamte Verdampferleistung beträgt 66.4 kW.

Die Kondensation des am Kopf der zweiten Reaktionskolonne entnommenen Dampfgemisches erfolgt 3-stufig und zwar in der 1. Stufe bei 174 - 165 °C (46 kW) der 2. Stufe bei 165 - 155 °C (17 kW) und der 3. Stufe bei 155 - 154 °C (1KW). Die Kondensationswärme der 1. und 2. Stufe wird zur Trennung eines Gemisches aus Dimethylcarbonat und Methanol verwendet.

Das Destillat der ersten Reaktionskolonne (486.6 kg/h) enthält 90.6 Gew.-% Dimethylcarbonat, 8.2 Gew.-% Methanol, 1 Gew.-% Phenol und 0.2 Gew.-% Anisol und wird zusammen mit einem weiteren Strom (36,6 kg/h) mit 97.3 Gew.-% Dimethylcarbonat und 2.7 Gew.-% Methanol einer zwei Destillationskolonnen enthaltenden Aufarbeitung zwecks Abtrennung des Methanols vom Dimethylcarbonat zugeführt.

Als Produkte der Methanolabtrennung werden 482 kg/h einer Dimethylcarbonat-Fraktion mit 98.75 Gew.-% Dimethylcarbonat, 1 Gew.-% Phenol, 0.2 Gew.-% Anisol und 0.05 Gew.-% Methanol und 41 kg/h einer Methanol-Fraktion mit 99.5 Gew.-% Methanol und 0.5 Gew.-% Dimethylcarbonat erhalten.

Da Methanol und Dimethylcarbonat ein Azeotrop bilden, wird die Trennung des Gemisches unter Anwendung des Zweidruckverfahrens durchgeführt. Dabei wird das Gemisch zunächst in einem Vorwärmer auf 137 °C erhitzt und dabei auch teilweise verdampft, anschließend in der ersten Destillationskolonne (K5) - auch als Dimethylcarbonat-Destillationskolonne bezeichnet - zunächst in die vorangehend genannte Dimethylcarbonat-Fraktion als Sumpfprodukt und eine Fraktion mit nahezu azeotroper Zusammensetzung (113.4 kg/h) mit 76.1 Gew.-% Methanol und 23.9 Gew.-% Dimethylcarbonat als Destillat zerlegt.

Die Dimethylcarbonat-Destillationskolonne arbeitet bei einem Kopfdruck von 5,5 bar und einem Rücklaufverhältnis von 1,2 und hat einen Verstärkungsteil mit 16 theoretischen Stufen und einen Abtriebsteil mit 7 theoretischen Stufen.

Dabei ergibt sich eine Temperatur im Sumpf der Kolonne von 154.2 °C. Die erforderliche Verdampfungswärme beträgt 59 kW. Die Verdampfung des Sumpfproduktes erfolgt in zwei Naturumlaufverdampfern, wobei der größte Teil der Wärme (46 kW) in einem Umlaufverdampfer, der gleichzeitig als erster Kondensator der zweiten Reaktionskolonne fungiert, ausgetauscht wird. Die restliche Verdampfungswärme wird in einem zweiten Umlaufverdampfer mittels Heizdampf bereitgestellt.

Der Wärmetauscher zur Vorwärmung des Zustroms der Dimethylcarbonat-Destillationskolonne fungiert gleichzeitig als zweiter Kondensator der zweiten Reaktionskolonne, wobei die übertragene Wärmemenge 17 kW beträgt.

In einer zweiten Destillationskolonne (K6) - auch als Methanol-Destillationskolonne bezeichnet - die bei einem Kopfdruck von 700 mbar und einen Rücklaufverhältnis von 2,3 arbeitet, wird Methanol als Sumpfprodukt (41 kg/h; MeOH/DMC 99,5/0,5 Gew.-%) abgetrennt. Das Destillat (72.3 kg/h) mit 62,4 Gew.-% Methanol und 37,6 Gew.-% Dimethylcarbonat wird erneut der Dimethylcarbonat-Destillationskolonne zugeführt.

Die Methanol-Destillationskolonne hat eine Trennleistung von 30 theoretischen Stufen, die sich zu gleichen Teilen auf Verstärkungs- und Abtriebsteil aufteilt.

Die im Verdampfer der Methanol-Destillationskolonne erforderliche Wärme (49 kW) wird durch die Kondensation der Dämpfe aus der Dimethylcarbonat-Destillationskolonne bereitgestellt. Der Kondensator der Dimethylcarbonat-Destillationskolonne fungiert somit gleichzeitig als Verdampfer der Methanol-Destillationskolonne.

Das Beispiel zeigt in eindrucksvoller Weise, wie der Energieverbrauch bei der Herstellung von Diphenylcarbonat durch effiziente Wärmeintegration deutlich reduziert werden kann.

So wird in der ersten Reaktionskolonne durch die Verwendung eines Zwischenkondensators der Wärmebedarf einschließlich der Erhitzung und Verdampfung der Edukte, Verdampfung im Sumpf der Kolonne und Beheizung der Reaktionszone von 183.3 auf 131.3 KW, also um 28.4 % reduziert. Gleichzeitig reduziert sich der Kühlmittelverbrauch von 183.2 auf 126.2 kW, somit um 31.1 %.

Durch die Wärmeintegration der zweiten Reaktionskolonne mit der Trennung des Methanol/Dimethylcarbonat-Gemisches wird der Heizmittelbedarf zur Trennung von Methanol und Dimethylcarbonat von 76 kW auf 13 kW also um 83 % reduziert werden. Gleichzeitig reduziert sich der Kühlmittelbedarf der zweiten Reaktionskolonne von 64 auf 1 KW also um 98,4 %.

### Beispiel 2 (erfindungsgemäß)

Unter sonst gleichen Bedingungen wie unter Beispiel 1, wird die erste Reaktionskolonne ohne Zwischenkondensator betrieben.

Durch die Wärmeintegration der zweiten Reaktionskolonne mit der Trennung des Methanol/Dimethylcarbonat-Gemisches kann der Heizmittelbedarf zur Trennung von Methanol und Dimethylcarbonat jedoch ebenfalls von 76 kW auf 13 kW also um 83 % reduziert werden. Gleichzeitig reduziert sich der Kühlmittelbedarf der zweiten Reaktionskolonne von 64 auf 1 KW also um 98,4 %.

Durch das erfindungsgemäße Verfahren ist folglich eine Einsparung erheblicher Energiemengen möglich.

## Patentansprüche

1. Verfahren zur Herstellung von wenigstens einem Diarylcarbonat und/oder Alkylarylcarbonat aus wenigstens einem Dialkylcarbonat und wenigstens einer aromatischen Hydroxyverbindung, wobei
(a) das oder die Dialkylcarbonat(e) in Gegenwart wenigstens eines Umesterungskatalysators mit der oder den aromatischen Hydroxyverbindung(en) in einer ersten Reaktionskolonne enthaltend wenigstens einen Verstärkungsteil im oberen Teil der Kolonne und wenigstens eine Reaktionszone unterhalb des Verstärkungsteils, welches mindestens zwei Sektionen aufweist, umgesetzt werden,
(b) der am Kopf der ersten Reaktionskolonne entnommene Dampf in wenigstens einem Kondensator ganz oder teilweise kondensiert wird,
(c) das Sumpfprodukt der ersten Reaktionskolonne wenigstens einer weiteren Reaktionskolonne enthaltend wenigstens einen Verstärkungsteil im oberen Teil der Kolonne und wenigstens eine Reaktionszone unterhalb des Verstärkungsteils zugeführt und in dieser (diesen) weiter umgesetzt wird,
(d) das in den Reaktionskolonnen nicht umgesetzte oder während der Reaktion gebildete Dialkylcarbonat ganz oder teilweise in wenigstens einem weiteren Verfahrensschritt enthaltend wenigstens eine Destillationskolonne vom während der Reaktion gebildeten Alkylalkohol getrennt, und
(e) das unter (d) abgetrennte Dialkylcarbonat wieder der ersten Reaktionskolonne zugeführt wird,
und die weitere(n) Reaktionskolonne(n) mit einem oder mehreren Kondensatoren ausgestattet sind,
wobei die durch Kondensation in diesen Kondensatoren gewonnene Kondensationswärme direkt oder indirekt wieder in das Verfahren zurückgeführt wird, **dadurch gekennzeichnet, dass** die durch Kondensation in dem oder den Kondensator(en) der weiteren Reaktionskolonne(n) gewonnene Kondensationswärme direkt oder indirekt, ganz oder teilweise zur Trennung des Dialkylcarbonats vom während der Reaktion gebildeten Alkylalkohols verwendet wird_und der Betriebsdruck in der oder den Destillationskolonne(n) des Verfahrensschritts zur Trennung von Dialkylcarbonat und Alkylalkohol so eingestellt wird, dass die Verdampfungstemperatur im Sumpf der Destillationskolonne(n) des Verfahrensschritts zur Trennung von Dialkylcarbonat und Alkylalkohol unter der Kondensationstemperatur in dem oder den Kondensator(en) der weiteren Reaktionskolonne(n) liegt.

2. Verfahren gemäß Anspruch 1, **dadurch gekennzeichnet, dass** die erste Reaktionskolonne einen Zwischenkondensator enthält und die in dem Zwischenkondensator gewonnene Kondensationswärme direkt oder indirekt, ganz oder teilweise zur Trennung des Dialkylcarbonats vom während der Reaktion gebildeten Alkylalkohol verwendet wird.

3. Verfahren gemäß Anspruch 1 oder 2, **dadurch gekennzeichnet, dass** wenigstens ein Verstärkungsteil der ersten Reaktionskolonne mit wenigstens einem Zwischenkondensator ausgestattet ist und die durch Kondensation in diesem Zwischenkondensator gewonnene Kondensationswärme direkt oder indirekt wieder in das Verfahren zurückgeführt wird.

4. Verfahren gemäß Anspruch 3, **dadurch gekennzeichnet, dass** der oder die Zwischenkondensator(en) in die erste Reaktionskolonne integriert oder als separate(r) Zwischenkondensator(en) außerhalb der Kolonne ausgeführt ist (sind).

5. Verfahren gemäß wenigstens einem der Ansprüche 1 bis 4, **dadurch gekennzeichnet, dass** der Verstärkungsteil der ersten Reaktionskolonne, der mit wenigstens einem Zwischenkondensator ausgestattet ist, in einen unteren und einen oberen Verstärkungsteil unterteilt ist, wovon sich der untere Verstärkungsteil unterhalb des Zwischenkondensators und der obere Verstärkungsteil oberhalb des Zwischenkondensators befindet.

6. Verfahren gemäß Anspruch 5, **dadurch gekennzeichnet, dass** der oder die Zwischenkondensator(en) und der obere Verstärkungsteil in die erste Reaktionskolonne integriert oder als separate Vorrichtung außerhalb der Kolonne ausgeführt sind.

7. Verfahren gemäß wenigstens einem der Ansprüche 1 bis 6, **dadurch gekennzeichnet, dass** es sich bei dem oder den Kondensatore(n) der weiteren Reaktionskolonne(n) um einen oder mehrere Kondensator(en) am Kopf der weiteren Reaktionskolonne(n) handelt.

8. Verfahren gemäß wenigstens einem der Ansprüche 1 bis 7, **dadurch gekennzeichnet, dass** die durch Kondensation in dem oder den Kondensator(en) der weiteren Reaktionskolonne(n) und/oder dem oder den gegebenenfalls vorhandenen Zwischenkondensator(en) der ersten Reaktionskolonne gewonnene Kondensationswärme direkt oder indirekt, teilweise zur Trennung des Dialkylcarbonats vom während der Reaktion gebildeten Alkylalkohols und teilweise zur Verdampfung des in die erste Reaktionskolonne eingeleiteten Dialkylcarbonats verwendet wird.

9. Verfahren gemäß wenigstens einem der Ansprüche 2 bis 8, **dadurch gekennzeichnet, dass** die durch Kondensation in dem oder den Kondensator(en) der weiteren Reaktionskolonne(n) gewonnene Kondensationswärme direkt oder indirekt, ganz oder teilweise zur Trennung des Dialkylcarbonats vom während der Reaktion gebildeten Alkylalkohols verwendet wird und die durch Kondensation in dem oder den Zwischenkondensator(en) der ersten Reaktionskolonne gewonnene Kondensationswärme direkt oder indirekt, ganz oder teilweise zur Verdampfung des in die erste Reaktionskolonne eingeleiteten Dialkylcarbonats verwendet wird.

10. Verfahren gemäß wenigstens einem der Ansprüche 1 bis 9, **dadurch gekennzeichnet, dass** die Temperatur der Reaktionszone der ersten Reaktionskolonne im Bereich von 100 bis 300°C, die Temperatur der Reaktionszone der weiteren Reaktionskolonne(n) im Bereich von 100 bis 300°C, der Druck der Reaktionszone der ersten Reaktionskolonne im Bereich von 0.5 bis 20 bar liegen und der Druck der Reaktionszone der weiteren Reaktionskolonne(n) im Bereich von 0,2 bis 10 bar liegen.

11. Verfahren gemäß wenigstens einem der Ansprüche 1 bis 10, **dadurch gekennzeichnet, dass** es sich bei dem Diarycarbonat um Diphenylcarbonat, bei dem Dialkylcarbonat um Dimethylcarbonat oder Diethylcarbonat und bei der aromatischen Hydroxyverbindung um Phenol handelt.

12. Verfahren gemäß wenigstens einem der Ansprüche 1 bis 11, **dadurch gekennzeichnet, dass** der am Kopf der ersten Reaktionskolonne entnommene Dampf ein Gemisch aus Dialkylcarbonat und während der Reaktion gebildetem Alkylalkohol enthält und nach Kondensation am Kopf der ersten Reaktionskolonne ganz oder teilweise wenigstens einem weiteren Verfahrensschritt zur Trennung von Dialkylcarbonat und Alkylalkohol zugeführt wird.

## Claims

1. Process for preparing at least one diaryl carbonate and/or alkyl aryl carbonate from at least one dialkyl carbonate and at least one aromatic hydroxyl compound, wherein
(a) the dialkyl carbonate(s) is/are reacted in the presence of at least one transesterification catalyst with the aromatic hydroxyl compound(s) in a first reaction column containing at least one rectifying section in the upper part of the column and at least one reaction zone below the rectifying section which has at least two sections,
(b) the vapour withdrawn at the top of the first reaction column is wholly or partly condensed in at least one condenser,
(c) the bottom product of the first reaction column is fed to at least one further reaction column containing at least one rectifying section in the upper part of the column and at least one reaction zone below the rectifying section and converted further therein,
(d) the dialkyl carbonate unconverted in the reaction columns or formed during the reaction is separated wholly or partly from the alkyl alcohol formed during the reaction in at least one further process step containing at least one distillation column, and
(e) the dialkyl carbonate removed under (d) is fed back to the first reaction column,
and the further reaction column(s) is/are equipped with one or more condensers,
wherein the heat of condensation obtained by condensation in these condensers is recycled directly or indirectly back into the process, **characterized in that** the heat of condensation obtained by condensation in the condenser(s) for the further reaction column(s) is used directly or indirectly, wholly or partly, for separation of the dialkyl carbonate from the alkyl alcohol formed during the reaction, and the operating pressure in the distillation column(s) in the process step for separation of dialkyl carbonate and alkyl alcohol is adjusted such that the evaporation temperature in the bottom of the distillation column(s) in the process step for separation of dialkyl carbonate and alkyl alcohol is below the condensation temperature in the condenser(s) for the further reaction column(s).

2. Process according to Claim 1, **characterized in that** the first reaction column contains an intermediate condenser and the heat of condensation obtained in the intermediate condenser is used directly or indirectly, wholly or partly, for separation of the dialkyl carbonate from the alkyl alcohol formed during the reaction.

3. Process according to Claim 1 or 2, **characterized in that** at least one rectifying section of the first reaction column is equipped with at least one intermediate condenser and the heat of condensation obtained by condensation in this intermediate condenser is recycled directly or indirectly back into the process.

4. Process according to Claim 3, **characterized in that** the intermediate condenser(s) is/are integrated into the first reaction column or executed as separate intermediate condenser(s) outside the column.

5. Process according to at least one of Claims 1 to 4, **characterized in that** the rectifying section equipped with at least one intermediate condenser in the first reaction column is divided into a lower and upper rectifying section, of which the lower rectifying section is below the intermediate condenser and the upper rectifying section above the intermediate condenser.

6. Process according to Claim 5, **characterized in that** the intermediate condenser(s) and the upper rectifying section are integrated into the first reaction column or executed as a separate apparatus outside the column.

7. Process according to at least one of Claims 1 to 6, **characterized in that** the condenser(s) for the further reaction column(s) is/are one or more condenser(s) at the top of the further reaction column(s).

8. Process according to at least one of Claims 1 to 7, **characterized in that** the heat of condensation obtained by condensation in the condenser(s) for the further reaction column(s) and/or any intermediate condenser(s) present for the first reaction column is used directly or indirectly, partly for separation of the dialkyl carbonate from the alkyl alcohol formed during the reaction and partly for evaporation of the dialkyl carbonate introduced into the first reaction column.

9. Process according to at least one of Claims 2 to 8, **characterized in that** the heat of condensation obtained by condensation in the condenser(s) for the further reaction column(s) is used directly or indirectly, wholly or partly, for separation of the dialkyl carbonate from the alkyl alcohol formed during the reaction, and the heat of condensation obtained by condensation in the intermediate condenser(s) for the first reaction column is used directly or indirectly, wholly or partly, for evaporation of the dialkyl carbonate introduced into the first reaction column.

10. Process according to at least one of Claims 1 to 9, **characterized in that** the temperature of the reaction zone of the first reaction column is in the range from 100 to 300°C, the temperature of the reaction zone of the further reaction column(s) is in the range from 100 to 300°C, the pressure of the reaction zone of the first reaction column is in the range from 0.5 to 20 bar and the pressure of the reaction zone of the further reaction column(s) is in the range from 0.2 to 10 bar.

11. Process according to at least one of Claims 1 to 10, **characterized in that** the diaryl carbonate is diphenyl carbonate, the dialkyl carbonate is dimethyl carbonate or diethyl carbonate, and the aromatic hydroxyl compound is phenol.

12. Process according to at least one of Claims 1 to 11, **characterized in that** the vapour withdrawn at the top of the first reaction column comprises a mixture of dialkyl carbonate and alkyl alcohol which has formed during the reaction and, after condensation at the top of the first reaction column, is fed wholly or partly to at least one further process step for separation of dialkyl carbonate and alkyl alcohol.

## Revendications

1. Procédé pour la préparation d'au moins un carbonate de diaryle et/ou carbonate d'alkylaryle à partir d'au moins un carbonate de dialkyle et d'au moins un composé aromatique à fonction hydroxy,
(a) le ou les carbonate(s) de dialkyle étant transformé(s) en présence d'au moins un catalyseur de transestérification avec le ou les composé(s) aromatique(s) à fonction hydroxy dans une première colonne de réaction contenant au moins une partie d'enrichissement dans la partie supérieure de la colonne et au moins une zone de réaction sous la partie d'enrichissement, qui présente au moins deux sections,
(b) la vapeur prélevée en tête de la première colonne de réaction étant condensée totalement ou partiellement dans au moins un condenseur,
(c) le produit de fond de la première colonne de réaction étant alimenté dans au moins une autre colonne de réaction contenant au moins une partie d'enrichissement dans la partie supérieure de la colonne et au moins une zone de réaction sous la partie d'enrichissement et étant transformé davantage dans celle(s)-ci,
(d) le carbonate de dialkyle non transformé ou formé pendant la réaction dans les colonnes de réaction étant séparé complètement ou partiellement dans au moins une autre étape de procédé, contenant au moins une colonne de distillation, de l'alcool alkylique formé pendant la réaction, et
(e) le carbonate de dialkyle séparé au point (d) étant à nouveau alimenté dans la première colonne de réaction,
et la ou les autre(s) colonne(s) de réaction étant équipée(s) d'un ou de plusieurs condenseurs,
la chaleur de condensation produite par la condensation dans ces condenseurs étant à nouveau recyclée directement ou indirectement dans le procédé, **caractérisé en ce que** la chaleur de condensation produite par la condensation dans le ou les condenseur(s) de l'autre/des autres colonne(s) de réaction est utilisée directement ou indirectement, totalement ou partiellement pour la séparation du carbonate de dialkyle de l'alcool alkylique formé pendant la réaction et la pression d'exploitation dans la/les colonne(s) de distillation de l'étape de procédé pour la séparation du carbonate de dialkyle et de l'alcool alkylique est réglée de manière telle que la température d'évaporation dans le fond de la/des colonne(s) de distillation de l'étape de procédé pour la séparation du carbonate de dialkyle et de l'alcool alkylique se situe sous la température de condensation dans le ou les condenseur(s) de l'autre/des autres colonne(s) de réaction.

2. Procédé selon la revendication 1, **caractérisé en ce que** la première colonne de réaction contient un condenseur intermédiaire et la chaleur de condensation produite dans le condenseur intermédiaire est utilisée directement ou indirectement, totalement ou partiellement, pour la séparation du carbonate de dialkyle de l'alcool alkylique formé pendant la réaction.

3. Procédé selon la revendication 1 ou 2, **caractérisé en ce qu'**au moins une partie d'enrichissement de la première colonne de réaction est équipée d'au moins un condenseur intermédiaire et la chaleur de condensation produite par condensation dans ce condenseur intermédiaire est à nouveau recyclée directement ou indirectement dans le procédé.

4. Procédé selon la revendication 3, **caractérisé en ce que** le ou les condenseur(s) intermédiaire(s) est/sont intégré(s) dans la première colonne de réaction ou réalisé(s) comme condenseur(s) intermédiaire(s) séparé(s) en dehors de la colonne.

5. Procédé selon au moins l'une quelconque des revendications 1 à 4, **caractérisé en ce que** la partie d'enrichissement de la première colonne de réaction, qui est équipée d'au moins un condenseur intermédiaire, est divisée en une partie d'enrichissement inférieure et une partie d'enrichissement supérieure, dont la partie d'enrichissement inférieure se trouve sous le condenseur intermédiaire et la partie d'enrichissement supérieure se trouve au-dessus du condenseur intermédiaire.

6. Procédé selon la revendication 5, **caractérisé en ce que** le ou les condenseur(s) intermédiaire(s) et la partie d'enrichissement supérieure sont intégrés dans la première colonne ou réalisés comme dispositifs séparés en dehors de la colonne.

7. Procédé selon au moins l'une quelconque des revendications 1 à 6, **caractérisé en ce qu'**il s'agit, pour le ou les condenseur(s) de l'autre/des autres colonne(s) de réaction, d'un ou de plusieurs condenseur(s) en tête de l'autre/des autres colonne(s) de réaction.

8. Procédé selon au moins l'une quelconque des revendications 1 à 7, **caractérisé en ce que** la chaleur de condensation produite par condensation dans le ou les condenseur(s) de l'autre/des autres colonne(s) de réaction et/ou le ou les condenseur(s) intermédiaire(s) le cas échéant présent(s) de la première colonne de réaction est utilisée directement ou indirectement, partiellement pour la séparation du carbonate de dialkyle de l'alcool alkylique formé pendant la réaction et partiellement pour l'évaporation du carbonate de dialkyle introduit dans la première colonne de réaction.

9. Procédé selon au moins l'une quelconque des revendications 2 à 8, **caractérisé en ce que** la chaleur de condensation produite par condensation dans le ou les condenseur(s) de l'autre/des autres colonne(s) de réaction est utilisée directement ou indirectement, totalement ou partiellement pour la séparation du carbonate de dialkyle de l'alcool alkylique formé pendant la réaction et la chaleur de condensation produite dans le ou les condenseur(s) intermédiaire(s) de la première colonne de réaction est utilisée directement ou indirectement, totalement ou partiellement pour l'évaporation du carbonate de dialkyle introduit dans la première colonne de réaction.

10. Procédé selon au moins l'une quelconque des revendications 1 à 9, **caractérisé en ce que** la température de la zone de réaction de la première colonne de réaction se situe dans la plage de 100 à 300°C, la température de la zone de réaction de l'autre/des autres colonne(s) de réaction se situe dans la plage de 100 à 300°C, la pression de la zone de réaction de la première colonne de réaction se situe dans la plage de 0,5 à 20 bars et la pression de la zone de réaction de l'autre/des autres colonne(s) de réaction se situe dans la plage de 0,2 à 10 bars.

11. Procédé selon au moins l'une quelconque des revendications 1 à 10, **caractérisé en ce qu'**il s'agit, pour le carbonate de diaryle, de carbonate de diphényle, pour le carbonate de dialkyle de carbonate de diméthyle ou de carbonate de diéthyle et pour le composé aromatique à fonction hydroxy de phénol.

12. Procédé selon au moins l'une quelconque des revendications 1 à 11, **caractérisé en ce que** la vapeur prélevée en tête de la première colonne de réaction contient un mélange de carbonate de dialkyle et d'alcool alkylique formé pendant la réaction et est alimentée après la condensation en tête de la première colonne de réaction, totalement au partiellement, dans au moins une autre étape de procédé pour la séparation du carbonate de dialkyle et de l'alcool alkylique.
